**Europäisches Patentamt**

**European Patent Office**

**Office européen des brevets**

(11) Publication number : **0 633 316 A1**

(12) **EUROPEAN PATENT APPLICATION**

(21) Application number : **94401497.6**

(22) Date of filing : **30.06.94**

(51) Int. Cl.⁶ : **C12N 15/57,** C12N 9/52, C12N 1/21, A23C 19/032, C12P 21/06

(30) Priority : **01.07.93 GB 9313586**

(43) Date of publication of application :
**11.01.95 Bulletin 95/02**

(84) Designated Contracting States :
**AT BE CH DE DK ES FR GB GR IE IT LI LU MC NL PT SE**

(71) Applicant : **EUROPEAN ECONOMIC COMMUNITY E.E.C.**
**Bâtiment Jean Monnet**
**Plateau du Kirchberg**
**L-2920 Luxembourg (LU)**

(72) Inventor : **Klein,Jürgen Robert**
**Mackenbacher Strasse 15**
**D-67685 Weilerbach (DE)**
Inventor : **Plapp,Roland**
**Am Harzhübel 88**
**D-67663 Kaiserslautern (DE)**

(74) Representative : **Grosset-Fournier, Chantal Catherine**
**Grosset-Fournier & Demachy s.a.r.l.**
**103 rue La Fayette**
**F-75010 Paris (FR)**

(54) **Lys-aminopeptidase PepN from lactobacillus delbruckii ssp. Lactis, nucleic acids coding for it, and its use in fermentation processes.**

㊗    The invention relates to a protein as produced by *Lactobacillus delbrückii* ssp. *lactis*, and capable of hydrolysing β-naphtylamides, or fragments thereof having this enzymatic activity.
   The invention relates also to the use of said protein or fragments thereof for the preparation of fermented foodstuff, and more particularly of cheese.

EP 0 633 316 A1

The invention relates to a protein, PepN, as produced by *Lactobacillus delbrückii* ssp. *lactis* and capable of hydrolysing β-naphtylamides, or polypeptides derived thereof, and more particularly recombinant PepN from said *Lactobacillus* strain or recombinant polypeptides derived thereof, said derived polypeptides having this enzymatic activity of hydrolysing β-naphtylamides.

The invention relates also to the use of said protein or polypeptides derived thereof in fermentation processes, such as processes for the preparation of fermented foodstuff, and more particularly cheese.

The invention also relates to processes for preparing the PepN from said *Lactobacillus* strain or said derived polypeptides, which are in a state of biological purity such that they can be used in fermentation processes.

The invention also relates to nucleic acids (or nucleotide sequences) coding for PepN from said *Lactobacillus* strain or said derived polypeptides, and to cellular hosts containing said nucleic acids and their use in fermentation processes.

Furthermore, the invention relates to fermentation processes, and more particularly to processes for the preparation of fermented foodstuff, and kits, using the PepN from said *Lactobacillus* strain or said derived polypeptides, and/or said cellular hosts.

By "recombinant polypeptides" it is to be understood that it relates to any molecule having a polypeptidic chain liable to be produced by genetic engineering, through transcription and translation of a corresponding DNA sequence under the control of appropriate regulation elements within a efficient cellular host. Consequently the expression "recombinant polypeptides" such as is used herein does not exclude the possibility for the polypeptides to comprise other groups, such as glycosylated groups.

The term "recombinant" indeed involves the fact that the polypeptide has been produced by genetic engineering, particularly because it results from the expression in a cellular host of the corresponding nucleic acid sequences which have previously been introduced into the expression vector used in said host.

Nevertheless, it must be understood that this expression does not exclude the possibility for the polypeptide to be produced by a different process, for instance by classical chemical synthesis according to methods used in the protein synthesis or by proteolytic cleavage of larger molecules.

The expression "biologically pure" or "biological purity" or "in a substantially pure form" means on the one hand a grade of purity such that the polypeptide or recombinant polypeptide can be used for the preparation of fermented foodstuff and on the other hand the absence of contaminants, more particularly of natural contaminants.

The lactobacilli used as starter cultures in the industrial dairy fermentation need to have an efficient proteolytic system. Since the concentration of free essential amino acids in milk are low, a variety of proteolytic enzymes is necessary for the breakdown of milk casein. By the combined action of proteinases and peptidases, milk protein is degraded to peptides and amino acids, which are required for cell growth and which contribute to the organoleptic properties of the foods. Also the flavour of milk products is primarily based on this degradation. The proteolytic cascade must be initiated extracellularly and successive coordinated degradation of peptides by endo- and exopeptidases, with cell wall and cytoplasmic localizations, generate amino acids necessary for growth. The importance of the proteolytic system for dairy product quality has resulted in an increased fundamental research of the enzymes and genes involved. Various genes and corresponding enzymes from lactococci strains have been investigated, but less is known about the proteolytic system of lactobacilli.

An aspect of the invention is to provide a new family of nucleic acids coding for new proteins and polypeptides which can be used in fermentation processes, and more particularly for the preparation of fermented foodstuff.

Another aspect of the invention is to provide cellular hosts transformed with said nucleic acids which can be used in fermentation processes, and more particularly as starter organisms in the fermentation of milk.

Another aspect of the invention is to provide nucleic acids coding for the peptidic chain of biologically pure recombinant polypeptides which enable their preparation on a large scale.

Another aspect of the invention is to provide new proteins and polypeptides which can be used in fermentation processes.

Another aspect of the invention is to provide new fermentation processes, such as processes for the preparation of fermented foodstuff, and more particularly cheese.

The invention relates to the cloning, expression, and nucleotide sequence of the lys-aminopeptidase gene (*pepN*) from *Lb. delbrückii* ssp. *lactis* and to some characteristics of the purified enzyme. The gene has been designated *pepN* since it complements an *E. coli pepN* mutation, and as reported hereafter, there is extensive amino acid homology to other aminopeptidases N (E.C. 3.4.11.2). The enzyme PepN is characterized by its specific action on β-naphtylamides.

The invention relates to a protein in a substantially pure form, as produced by *Lactobacillus delbrückii* ssp. *lactis*, said protein, also called PepN, being capable of hydrolysing β-naphtylamides, and more particularly lysine-β-naphtylamide, or fragments thereof having this enzymatic activity (i.e. this hydrolysing amino acid β-naphtylamides activity).

By hydrolysing amino acid β-naphtylamides, one should understand that such activity corresponds to aminopeptidase N (PepN); see Table 3 hereafter.

The invention relates more particularly to the PepN protein such as described above and produced by *Lactobacillus delbrückii* ssp. *lactis* WS87, deposited at the Deutsche Sammlung von Mikroorganismen (DSM) under the number 7290 on October 15, 1992.

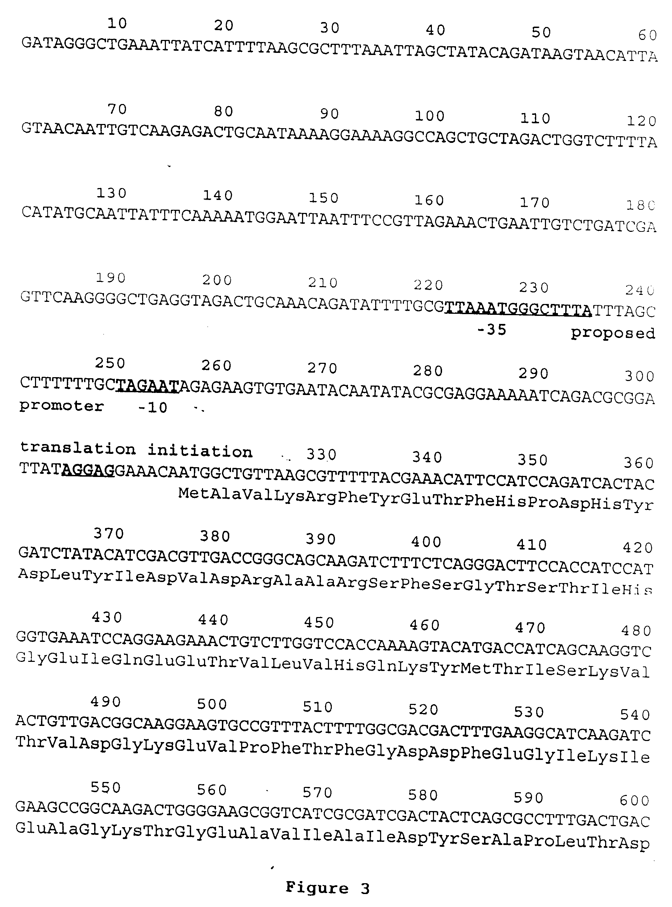

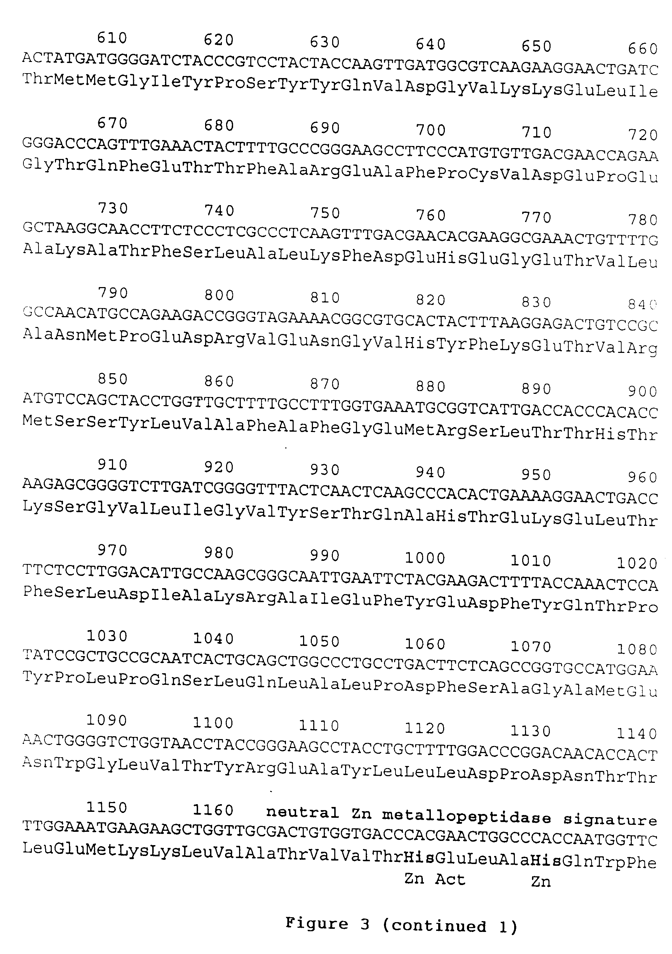

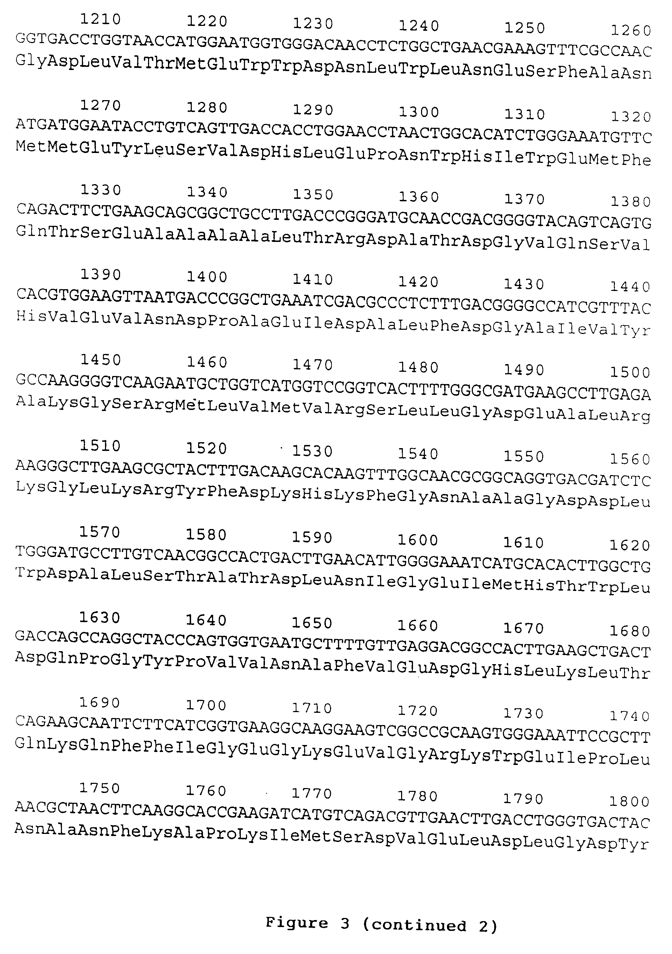

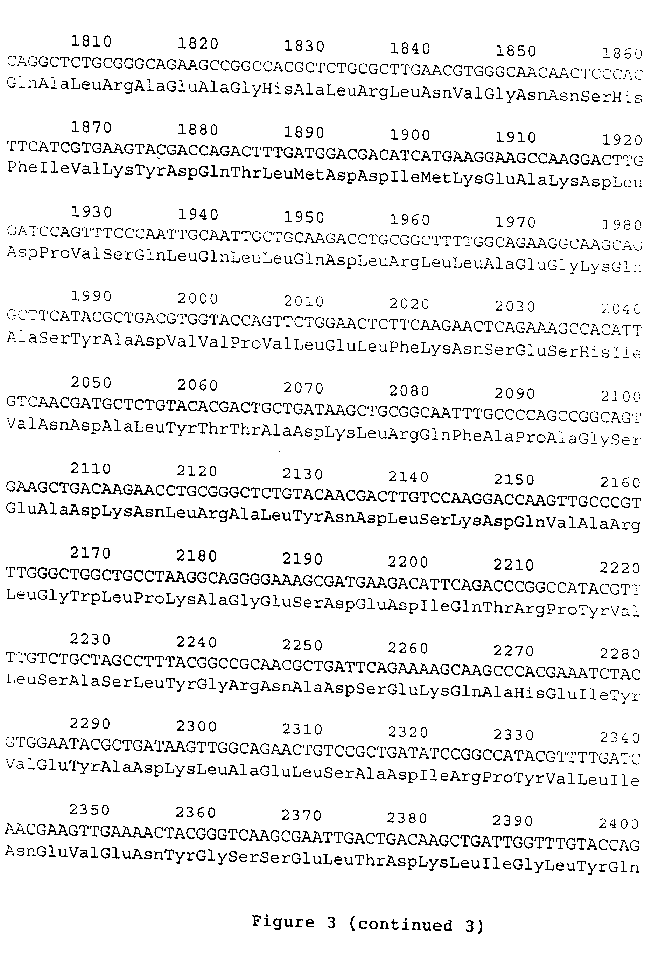

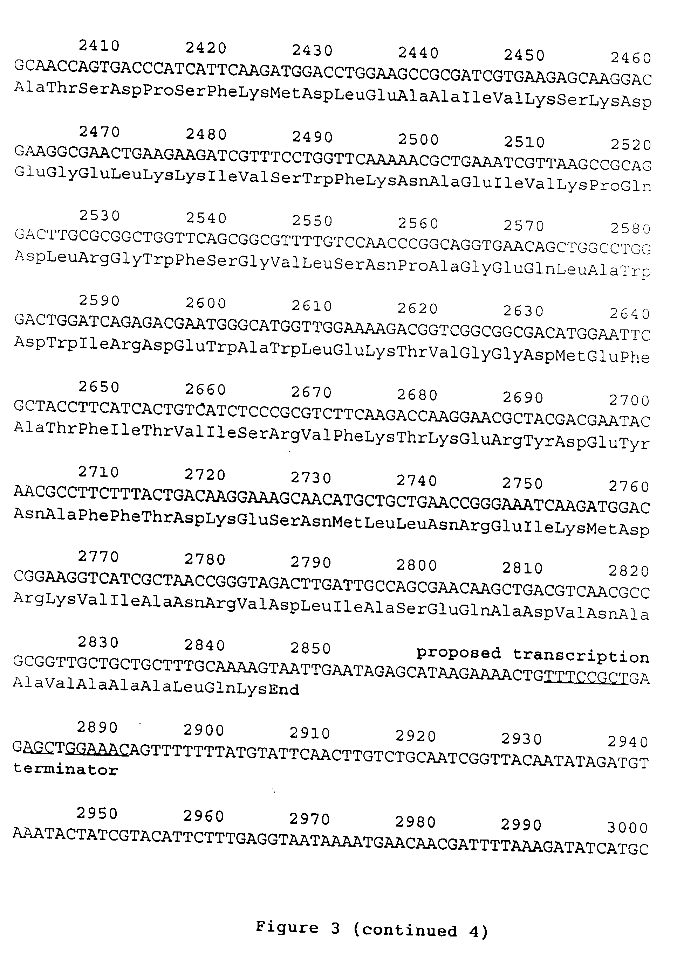

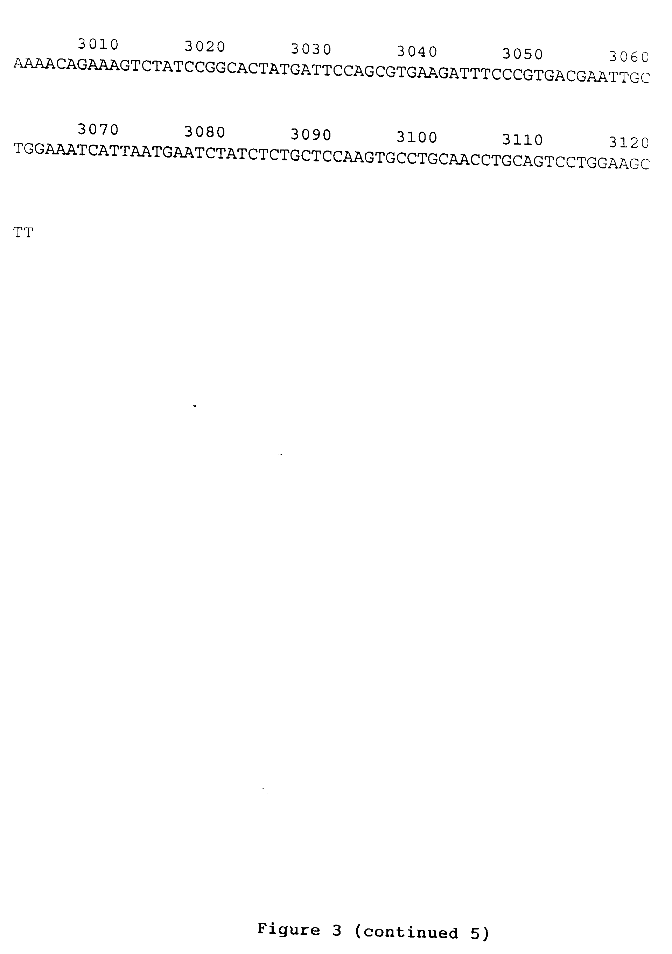

The invention relates more particularly to the PepN protein represented on figure 3, and by SEQ ID NO 2, or polypeptides derived thereof, such as fragments or muteins (which differ from said protein by addition and/or substitution and/or suppression of one or several amino acid) thereof, provided that said derived polypeptides are capable of hydrolysing β-naphtylamides.

Said PepN represented by SEQ ID NO 2, is more particularly characterized in that:
- its isoelectric point calculated from the nucleotide sequence represented by SEQ ID NO 1, is 4,48, and its isoelectric point determined by preparative isoelectric focusing after purification of said protein, is 4,2,
- its molecular weight calculated from the nucleotide sequence represented by SEQ ID NO 1, is 95,358 kDa, and its molecular weight determined after purification, is 95 kDa,
- its specific chromogenic substrates are chosen among those listed in Table 3,
- its specific inhibitors are the following:
    . EDTA: end concentration 1 mM: 3 % relative activity,
    . Phenanthroline: end concentration 1 mM: 4 % relative activity.

The percentages of relative activity indicated above, correspond to residual activities after addition of the inhibitor as compared with the control without inhibitor. The conditions and methods for determination are taken from "Proteolytic enzymes, a practical approach, Beynon R.J. and Bond J.S., Oxford University Press 1989." (Chapter 4, Determination of protease mechanism, Ben M. Dunn).

The PepN according to the invention is more particularly characterized in that it is obtained in a purified state, from a cell extract of said *Lactobacillus delbrückii* ssp. *lactis* WS87 by the following procedure:
- fractionation of the cell extract by salting out at 4°C with streptomycin sulfate,
- centrifugation,
- anion exchange chromatography by applying the supernatant obtained at the previous step to an appropriate column, such as a column of Q-Sepharose Fast Flow (Pharmacia), and pooling the eluted fractions having the highest specific activity against L-Pro-p-nitroanilide,
- preparative isoelectric focusing, by applying the pooled fractions obtained at the previous step to an appropriate column, such as a LKB column (Types 8100-1), with ampholytes within a range of pH 4 to 6, and pooling the eluted fractions having the highest specific activity against Lys-paranitroanilide.

The invention also relates to nucleic acids coding for a protein according to the invention, i.e. for the PepN protein or plypeptides derived thereof as described above.

The invention relates more particularly to nucleic acids characterized in that:
- they comprise all or part of the nucleic acid represented on figure 3, and by SEQ ID NO 1, coding for a protein according to the invention, or its complementary sequence,
- or they hybridize with all or part of said nucleic acid represented by SEQ ID NO 1, or with its complementary sequence.

The invention relates more particularly to a nucleic acid coding for the PepN protein represented by SEQ ID NO 2, or for polypeptides derived thereof having said hydrolysing β-naphtylamides activity, said nucleic acid comprising all or part of the nucleotide sequence delimited by the nucleotide located in position 316 and the nucleotide located in position 2844 in the nucleotide sequence represented by SEQ ID NO 1.

The invention also relates to any nucleic acid susceptible to hybridize with all or part of nucleic acids such as described above, and more particularly to any nucleic acid derived from this latter according to the degeneracy of the genetic code, and more particularly by substitution, and/or addition, and/or suppression of one or several nucleotides of said nucleic acids described above, said derived nucleic acid being able to code for the PepN protein represented by SEQ ID NO 2, or for polypeptides derived thereof such as defined above.

By way of illustration, the hybridization above-mentioned can be performed with membranes like Heybond™-N (Amersham) or Hybridization Transfer Membrane (Micron Separations Inc.) under conditions as described by the supplier.

The invention also relates to recombinant nucleic acid containing at least one of the nucleic acids according to the invention, inserted in a heterologous nucleic acid.

The invention also relates to recombinant vectors comprising a vector sequence, notably of the type plasmid (such as plasmids originating from lactic acid bacteria), cosmid or phage, and a nucleic acid described above, in one of the non-essential sites for its replication, and optionally one or several nucleic acid(s) coding for other aminopeptidases such as PepX (Meyer-Barton et al., 1993).

Prefered recombinant vector contains in one of its non essential sites for its replication necessary elements to promote the expression of polypeptides according to the invention, in a cellular host and possibly a promoter recognized by the polymerase of the cellular host, particularly an inducible promoter and possibly a signal sequence and/or an anchoring sequence.

The invention relates more particularly to recombinant vector, such as described above, containing the elements enabling the expression by *E. coli* or lactic acid bacteria including *lactococcus* and, especially, lactobacilli of the thermophilic group, of a nucleic acid according to the invention, inserted in the vectors.

The invention relates more particularly to recombinant vector, such as described above, constructed from replicons isolated from lactobacilli, especially from thermophilic *Lactobacillus* species.

The invention also relates to cellular hosts which are transformed by a recombinant vector according to the invention, and comprising the regulation elements enabling the expression of the nucleotide sequence coding for a protein according to the invention, and optionally, other aminopeptidases such as PepX, in these hosts.

The invention relates more particularly to cellular hosts, such as described above, chosen from among bacteria such as *E.coli*, or lactic acid bacteria including *lactococcus* and, especially, lactobacilli of the thermophilic group, transformed by a vector according to the invention.

From the nucleic acids of the invention, probes (i.e. cloned or synthetic oligonucleotides) can be inferred.

These probes can be from 25 nucleotides up to the total length of the *pepN* gene. These oligonucleotides can also be used as amplification primers in the PCR technique (PCR, Mullis and Faloona, Methods in Enzymology, vol. 155, p. 335, 1987) to generate specific enzymatically amplified fragments and/or as probes to detect fragments amplified between bracketing oligonucleotide primers.

The invention also relates to the expression products of a nucleic acid expressed by a transformed cellular host according to the invention.

The invention also relates to a process for preparing the PepN protein such as described above, comprising the following steps:

- the culture in an appropriate medium of a cellular host according to the invention,
- the recovery of the polypeptide produced by the above said cellular host from the above said culture medium,
- the purification of the protein thus obtained, more particularly according to the method described above.

The invention also relates to fermentation processes comprising a step of treatment of material to be fermented with:

- an appropriate amount of the PepN protein or polypeptides derived thereof according to the invention, and optionally other proteases, and more particularly aminopeptidases such as PepX, and/or
- an appropriate amount of at least one of the transformed cellular host such as described above, comprising a nucleotide sequence coding for PepN, and optionally for other proteases, and more particularly aminopeptidases such as PepX,
- and optionally, an appropriate amount of lactobacilli, such as *Lactobacillus delbrückii* ssp. *lactis*.

The invention also relates to a process for the preparation of fermented foodstuff, and more particularly of cheese, which comprises a step of treatment of food material to be fermented, such as milk, with:

- an appropriate amount of the PepN protein polypeptides derived thereof according to the invention, and optionally other proteases, and more particularly aminopeptidases such as PepX,
- an appropriate amount of at least one of the transformed cellular host such as described above, comprising a nucleotide sequence coding for PepN, and optionally for other proteases, and more particularly aminopeptidases such as PepX,
- and optionally, an appropriate amount of lactobacilli, such as *Lactobacillus delbrückii* ssp. *lactis*.

The process for the preparation of fermented foodstuff described above can also comprise a step of treatment of food material to be fermented, such as milk, with other species and strains susceptible to be used as starter organisms in fermentation processes, and more particularly lactic acid bacteria susceptible to produce a PepX protein, such as *Lactococcus*, *Streptococcus* and *Lactobacillus*.

The fermentation processes according to the invention, are more particularly characterized in that they can be used as fermentation processes for the obtention of hard cheeses, such as Emmentaler type cheese (South Germany, Switzerland).

The invention also relates to foodstuff, and more particularly cheeses, such as obtained by fermentation processes as described above.

The invention also relates to the use of all or part of nucleotide sequences described above, as tools for analytical purpose. In that respect, the invention relates more particularly to the use of the above-mentioned nucleotide sequences, and more particularly all or part of the SEQ ID NO 1 sequence, in procedures for amino acid sequencing of peptides and production of chemical or pharmaceutical compounds in which the hydrolysis of β-naphtylamide residues is a required step.

The invention relates more particularly to the use of the polypeptides described above for the determination of the exact localization of PepN in *Lactobacillus delbrückii* ssp. *lactis*, rendering possible experiments performed by immunoblotting after cell fractionation and by electron microscopy of immunogold labelled peptidases.

The polypeptides of the invention can also be prepared according to the classical techniques in the field of peptide synthesis.

The synthesis can be carried out in homogeneous solution or in solid phase.

For instance, the synthesis technique in homogeneous solution which can be used is the one described by Houbenweyl in the book entitled "Methoden der organischen Chemie" (Method of organic chemistry) edited by E. Wünsch, vol. 15-I and II, THIEME, Stuttgart 1974.

The polypeptides of the invention can also be prepared in solid phase according to the methods described by Atherton and Sheppard in their book entitled "Solid phase peptide synthesis" (IRL Press, Oxford, New York, Tokyo, 1989).

The invention also relates to antibodies themselves formed against the PepN protein or polypeptides derived thereof according to the invention, more particularly by immunization of appropriate animals with said polypeptides and recovery of antibodies thus formed.

It goes without saying that this production is not limited to polyclonal antibodies.

It also relates to any monoclonal antibody produced by any hybridoma liable to be formed according to classical methods from splenic cells of an animal, particularly of a mouse or a rat, immunized against the purified polypeptide of the invention on the one hand, and of cells of a myeloma cell line on the other hand, and to be selected by its ability to produce the monoclonal antibodies recognizing the polypeptide which has been initially used for the immunization of the animals.

The invention also relates to any antibody of the invention labelled by an appropriate label of the enzymatic, fluorescent or radioactive type.

The invention also relates to the use of such antibodies, for example for the detection of the PepN protein or polypeptides derived thereof, as described above.

The invention also relates to a process for preparing the nucleic acids according to the invention.

A suitable method for chemically preparing the single-stranded nucleic acids (containing at most 100 nucleotides of the invention) can be carried out according to the automatic β-cyanoethyl phosphoramidite method of DNA synthesis described in Bioorganic Chemistry 4; 274-325 (1986).

In the case of single-stranded DNA, the material which is obtained at the end of the DNA synthesis can be used as such.

A suitable method for chemically preparing the double-stranded nucleic acids (containing at most 100 bp of the invention) comprises the following steps:

- DNA synthesis of one sense oligonucleotide using the automatic b-cyanoethyl phosphoramidite method above cited, and DNA synthesis of one antisense oligonucleotide using either the above-mentioned automatic b-cyanoethyl phosphoramidite method, or enzymatic transcription of the sense-strand using a specific primer hybridizing to the 3'-end of the sense strand,
- combining the sense and antisense oligonucleotide by hybridization in order to form a DNA duplex,
- cloning the DNA duplex obtained into a suitable plasmid vector and recovery of the DNA according to classical methods such as restriction enzyme digestion and agarose electrophoresis, or by PCR amplification according to the procedure outlined above.

A method for the chemical preparation of nucleic acids with lengths greater than 100 nucleotides - or base pairs, in the case of double-stranded nucleic acids - comprises the following steps:

- assembling the synthesized oligonucleotides, provided at their ends with different restriction sites, the sequences of which are compatible with the succession of amino acids in the natural peptide, according to the principle described by Urdea et al. in Proc. Nat. Acad. Sci. USA 80; 7461-7465 (1983),
- cloning the DNA thereby obtained into a suitable plasmid vector and recovery of the desired nucleic acid according to classical methods such as restriction enzyme digestion and agarose gel electrophoresis.

Other characteristics and advantages of the invention will appear in the following examples and the figures illustrating the invention.

In cell extracts of *Lactobacillus delbrückii* ssp. *lactis* DSM7290 a peptidase with the activity to hydrolyse phe-β-naphthylamide and his-β-naphthylamide could be detected. *Escherichia coli* lacking the enzyme activity in a enzymatic plate assay, which was based on the hydrolysis of these β-naphthylamide (β-NA) substrates, was used to screen high copy and low copy number plasmid libraries of size fractionated *Lactobacillus* DNA. Clones with the desired phenotype were detected, and the gene, designated *pepN*, was further subcloned and sequenced. A large open reading frame of 2529 nt is predicted to encode a protein of 843 amino acids (95358 Da). Analysis of the *pepN* sequence indicated that the enzyme is not subjected to posttranslational modification or exported via processing of a signal peptide. Comparison of the *pepN* gene from *Lb. delbrückii* ssp. *lactis* DSM7290 indicates that it is homologous to genes of the family of $Zn^{2+}$-metallohydrolases and shows identity with the active centre $Zn^{2+}$-binding motif of these enzymes. Like in all other organisms the substrate lys-$\beta$-NA is more effectively cleaved than phe- or his-$\beta$-NA's, used for screening in *E. coli*. The cloned enzyme was extremely overexpressed in *E. coli* and subclonig of the gene in *Lactobacillus casei* resulted in a moderate overexpression of approximately 20-fold. The cloned enzyme was purified from the *pepN* deficient *E. coli* strain CM89, using the substrate lys-p-nitroanilide. In a four step procedure including streptomycin sulfate precipitation, anion exchange chromatography, and gelfiltration the peptidase was purified to electrophoretic homogeneity.

The structural proteinase genes (*prtP: Kok et al., 1988; Kiwaki et al., 1989; Vos et al., 1989a; prtM: Haandrickman et al., 1989; Kiwaki et al., 1989; Vos et al., 1989b;*), the cysteine aminopeptidase (*pepC,* Chapot-Chartier et al., 1993), the X-prolyl-dipeptidyl-aminopeptidase genes (*pepX*; Nardi et al., 1991, Mayo et al., 1991), and the general aminopeptidase *pepN* (Stroman, 1992; van Alen-Boerrigter et al., 1991;) of a number of lactococci strains have been cloned and sequenced. From *Lactobacillus* strains only the proteinase genes (Holck and Naes, 1992). From *Lb. paracasei* and the X-prolyl-dipeptidyl-aminopeptidase gene from *Lb. delbrückii* ssp. *lactis* DSM7290 (Meyer et al., 1993), have been cloned and sequenced. *Lb. delbrückii* ssp. *lactis* is used as starter culture in the fermentation of swiss type cheese. Strain DSM7290, originally designated WS87, isolated from Emmenthaler cheese was chosen for screening of peptidase genes.

It was screened for complementation and expression in *E. coli*, with subtrates cleaved in cell extracts of DSM7290, but reacting negative in the enzyme plate assay used for screening. It has been decided to screen for heterologous expression in *E. coli* because of still moderate transformation efficiencies in lactobacilli and since peptidase activities in lactobacillal colonies cannot be reliably detected as a reason of acidification of agar plates and rigidity of the cell envelope.

I) Material and methods

Bacterial Strains, Plasmids, and Growth Conditions

The bacterial strains and plasmids used are summarized in Table 1. *Escherichia coli* was grown at 37°C in Luria broth (Miller, 1972), and lactobacilli in MRS (De Man et al., 1960) at 37°C. Ampicillin and kanamycin were added to concentrations of 100 and 40 µg/ml, respectively.

Transformations

*Lb. casei* LK1 (Zink et al., 1991) and *E. coli* cells (Dower et al., 1988) were transformed by electroporation using a Bio-Rad Gene Pulser (Bio-Rad Laboratories, Richmond, CA) as described before.

Recombinant DNA Techniques.

Restriction enzymes and other nucleic acid-modifying enzymes were used as recommended by the manufacturers. Isolation of plasmid DNA from *E. coli* was performed as described by Sambrock et al. (1989). Plasmid DNA isolation from *Lb. casei* or *Lb. curvatus* was performed as described elsewhere (Zink et al., 1991).

Isolation of chromosomal DNA from DSM7290

400 ml of prewarmed MRS medium was inoculated with 40 ml of an overnight culture of *Lb. delbrückii* ssp. *lactis* DSM7290 and incubated for 2.5 hours at 37°C. Cells were pelleted by centrifugation, washed once with 50 mM Tris-HCl pH 8, resuspended in 37,5 ml of sucrose 6.5 %, 50 mM Tris-HCl pH 8, 1 mM EDTA, 200 mg of Lysozym, and 2500 U of mutanolysin (Sigma) and incubated for 1 hour at 37°C. 3.75 ml of 0.25 M EDTA in 50 mM Tris-HCl pH 8 was added. Cells were lysed by the addition of 2.25 ml 20 % SDS. The lysed cells were subjected to proteinase K digestion at a concentration of 50 µg/ml, for 15 min at 50°C and 30 min at 60°C. EtBr was added to a concentration of 1 mg/ml and CsCl to reach a final density of 1.55 g/ml. Centrifugation to equilibrium and further purification of chromosomal DNA was performed as described by Sambrock et al. (1989).

Molecular cloning of the *Lb. delbrückii* ssp. *lactis pepN* gene

Construction of plasmid libraries:

Genomic plasmid libraries of *Lb. delbrückii* ssp. *lactis* DSM7290 were prepared in *E. coli* ER1562 using the positive selection vector pUH84 (Henrich and Plapp, 1984) and because overexpression of peptidase genes from this high copy number vector may be lethal to the host a second plasmid bank was constructed with vector pLG339 having only 6-8 copies per chromosome (Stoker et al., 1982). Partial *Sau*3A fragments of total DSM7290 DNA were size fractionated (10-40 % sucrose gradient) and 4-10 kb fragments were ligated into the dephosphorylated *Bam*HI sites of the vector molecules.

Identification of peptidase genes:

Colonies of *E. coli* transformed with the gene banks were screened for PepN activity by a plate staining method of Miller and Mackinnon (1974), recently used by Nardi et al. (1991). If chromogenic β-NA substrates are cleaved, reaction of the β-napthylamines with fast garnet CBC (Sigma), can be monitored by the formation of a red, non-diffusible azo dye.

DNA Sequence Analysis

For nucleotide sequencing of inserts in pLG339, a pair of universal sequencing primers, adjacent to the *Bam*HI cloning site was synthesized. Synthetic oligonucleotide primers deduced from the investigated sequence, were synthesized and allowed direct sequencing of double stranded plasmid DNA. The DNA sequence of each strand was determined using the T7 DNA polymerase sequencing kit (Pharmacia, Uppsala, Sweden), which is based on the dideoxynucleotide chain termination method (Sanger et al., 1977) in the presence of [$\alpha$-$^{35}$S]dATP (Amersham). For computer-assisted sequence analysis the Microgenie (Beckman, Palo Alto, CA), PC-Gene (IntelliGenetics, East El Camino Real, CA), and HUSAR (GENIUSnet, Heidelberg) software were used.

Sequencing primers were synthesized using Applied Biosystems Model 392 DNA synthesizer and reagents.

Preparation of cell extracts

Cell pellets of *E. coli* from a 2 l overnight culture were washed with 50 mM Tris-HCl pH 8.0, pelleted by centrifugation and resuspended in 20 ml of the same buffer. The bacteria were sonicated on ice (Bandelin sonifier; Sonopuls HD60) until more than 90 % of the cells were broken. Cell debris were removed by centrifugation at 52,000 g and 4°C for 60 min.

Lactobacillal cell extracts were prepared with 50 ml cultures and the modification that prior sonification of the more rigid bacteria, glass beads (diameter, 0.17-0.18 mm) were added to the cell pellets (2 Vol glassbeads/ 1 Vol cells). Centrifugation was performed in a Hereus Biofuge RS28 at 51,000 g and 4°C for 60 min.

The supernatants of both preparations contained approximately 50 mg/ml of protein as determined by the method of Lowry (1951).

Enzyme assay and effects of various chemical reagents in crude cell extracts

For characterization of the enzyme the p-nitroanilide subtrates (Bachem) were dissolved in water and added to the reaction mixture [Tris-HCl 10 mM, pH 8.0, and varying amounts of purified enzyme (1-200 ng)] to a concentration of 1 mM in a volume of 250 μl. Release of p-nitroaniline was measured after a 10 min incubation at 37°C at 405 mm in a LKB Ultrospec plus spectrophotometer. For rapid screening of active fractions during purification of the enzyme, samples were prepared in micro titer plates and measured in a BIORAD model 2550 EIA reader.

To study the mechanism of enzyme action, the inhibitors phenylmethansulphonyl fluoride (PMSF) at a concentration of 1 mM, pepstatin A at 1 μg/ml, L-trans-epoxysuccinyl-leucylamide(4-guanidino)-butane (E-64) at 0.1 mM, and 1,10-phenanthroline or EDTA at 1 mM were added to the enzyme and incubated for 30 min at 37°C. The substrate lys-p-nitroanilide was added and activity was measured spectrophotometrically by the release of p-nitroaniline.

Isoelectric focusing

Isoelectric focusing was carried out in a LKB column (type 8100-1) with ampholytes (Serva) ranging from pH 4 to 6. The pH gradient was stabilized by a glycerol gradient.

Purification

Step 1: Streptomycin sulfate precipitation

At 4°C a solution of 10 % streptomycin sulfate was slowly added to cell extract while stirring, to reach a final concentration of 2 %. The precipitate was discarded after centrifugation (10 min, 20,000 g.)

Steps 2 and 3: Anion exchange chromatography

The supernatant of the streptomycin sulfate precipitation was concentrated by passage through a PM30 Diaflow membrane (Amicon) and applied to a 38 by 2.2 cm column of Q-sepharose fast flow (Pharmacia) equilibrated with 0.1 M NaCl in 20 mM Tris-HCl pH 8.0 at 4°C. Proteins were eluted with a linear NaCl gradient of 0 to 0.5 M NaCl respectively in the same buffer. In a second passage a gradient ranging form 0.4 to 0.7 M NaCl was chosen. 4 ml fractions were collected at a flow rate of 150 ml/h and those with high PepN activity were pooled and concentrated by passage through a PM30 Diaflow membrane.

Step 4: Gelfiltration.

The enriched PepN preparation from anion exchange chromatography was applied to a 55 by 2.2 cm column of Fractogel TSK HW-55(S) (Merck) equilibrated with 20 mM Tris-HCl pH 7.2 at 4°C. Elution was performed with the same buffer at 20 ml/min and 2 ml fractions were collected.

II) Results and Discussion

Cloning of the *Lb. delbrückii* ssp. *lactis* DSM7290 *pepN* gene.

The plasmid libraries of DSM7290 chromosomal DNA in pUH84 and pLG339 respectively were transformed into *E. coli* ER1562. The *pepN* gene could be isolated by screening the colonies for peptolytic activities using the chromogenic substrates his-β-NA and phe-β-NA. In the plate assay these substrates were not cleaved by the homologous PepN of the *E. coli* host strain, and additionally they were not cleaved by *E. coli* harbouring the recently cloned genes for *pepX* (X-prolyl-dipeptidyl aminopeptidase) (Meyer et al., 1993) and *pepP* (prolin iminopeptidase, unpublished) from *Lb. delbrückii* ssp. *lactis* DSM7290. The gene has been designated *pepN* since sequence analysis revealed extensive amino acid homology to other aminopeptidases N (E.C. 3.4.11.2).

From approximately 50,000 colonies harbouring high copy number genebank plasmids no positive reaction could be detected in the plate assay, but in transformants with the low copy number plasmid bank, with pLG339 as vector, 3 his-β-NA and 3 phe-β-NA cleaving colonies out of 21,000 could be detected. The inability to identify positive clones in the pUH84 genebank indicated that overexpression of the peptidase gene is lethal for *E. coli*. Restriction analysis revealed that all plasmids isolated had insert sizes ranging from 5.2 to 8.0 kb, with a conserved identical core region. This agreed with the observation that all plasmids coded for an enzyme cleaving both substrates his-β-NA and phe-β-NA.

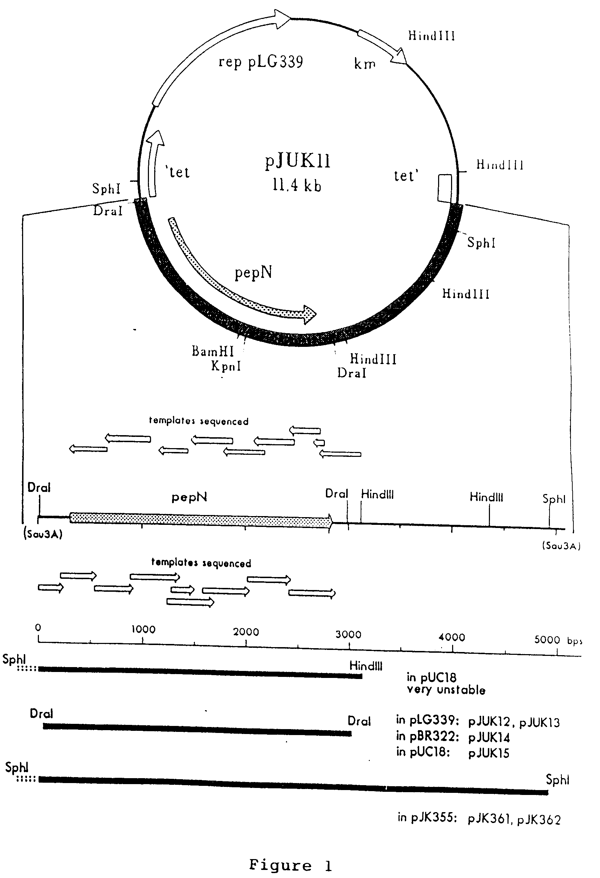

The plasmid pJUK11 with the smallest insert size of 5.2 kb was subject for further experiments (FIG.1). The chromogenic β-NA substrates have been used for colony screening and the analogous p-nitroanilides for characterization of the enzyme.

Plasmid encoded proteins

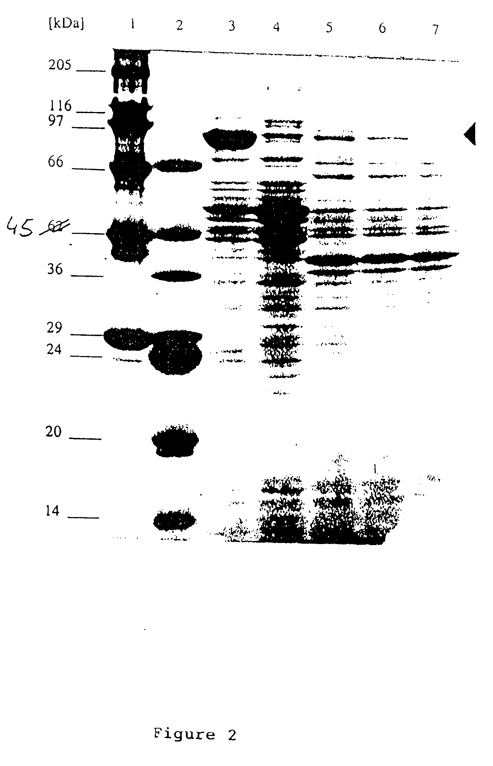

*E. coli* cells harbouring pJUK11 were sonified and the crude cell extracts subjected to SDS-PAGE (sodium dodecyl sulfate polyacrylamide gel electrophoresis) (Laemmli, 1970). A major 95 kDa protein appeared in ER-1562(pJUK11) extracts which constituted at least 50 % of the cytoplasmic *E. coli* proteins (FIG.2). This remarkable high level expression of *pepN* might be the reason for its lethality if cloned in a high copy number plasmid.

## Subcloning and nucleotide sequence analysis

Prior to sequence analysis the region coding for *pepN* was localized, calculating that approximately 2.6 kb should be sufficient to code for the 95 kDa protein detected by SDS-PAGE. Subcloning of a 3.2 kb *SphI/Hind*III fragment from pJUK11 in pUC18 (FIG.1) resulted in a plasmid coding for the *pepN* gene with expression of enzyme activity, but which was extremely unstable. Nevertheless it offered the possibility to determine the DNA sequence starting at the HindIII site with the universal primer of vector pUC18. The second strand, which is coding for *pepN* was sequenced using a synthesized 20mer primer, complementary to the tetracyclin resistance gene in pJUK11. The complete nucleotide sequence of both strands of the *SphI/Hind*III fragment was determined using synthetic oligonucleotide primers deduced from the investigated sequences. The lactobacillal nucleotide sequence determined (3122 bp, 49.4% C+G) contained one single open reading frame (FIG.3). Starting with a putative ATG codon it would code for 843 amino acids corresponding to a protein with a molecular weight of 95358 Da. This value corresponds well with the molecular weight of the enzyme predicted by SDS-PAGE.

## Transcription of the *pepN* gene

Upstream of the ATG start codon spaced by six base pairs, a putative ribosome binding site with a hexanucleotide stretch (AGGAGG) complementary to the *E. coli* 16 S-rRNA, and sequences that resemble promoter -10 and -35 sequences (Fig. 3) elements of gram positives (Van de Guchte et al., 1992) can be located. Currently we are going to compare the functionality of these sequences in *E. coli* and *Lb. delbrückii* ssp. *lactis*. The *pepN* coding sequence is followed by an inverted repeat which may form a stem-loop structure with a dG of -12.8 kcal/mol, and which may function as a transcriptional terminator, as predicted by the computer algorithm of Brendel and Trifonov (1984).

## Comparison of the lactobacillal PepN sequence to aminopeptidases of other species

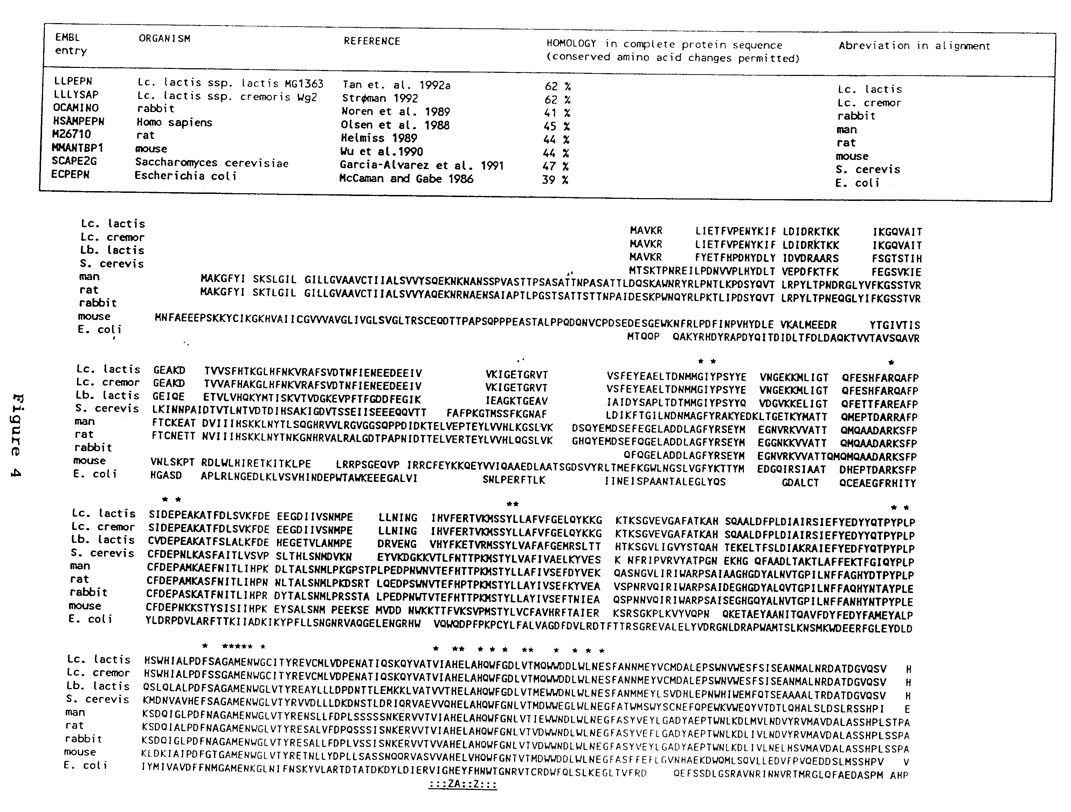

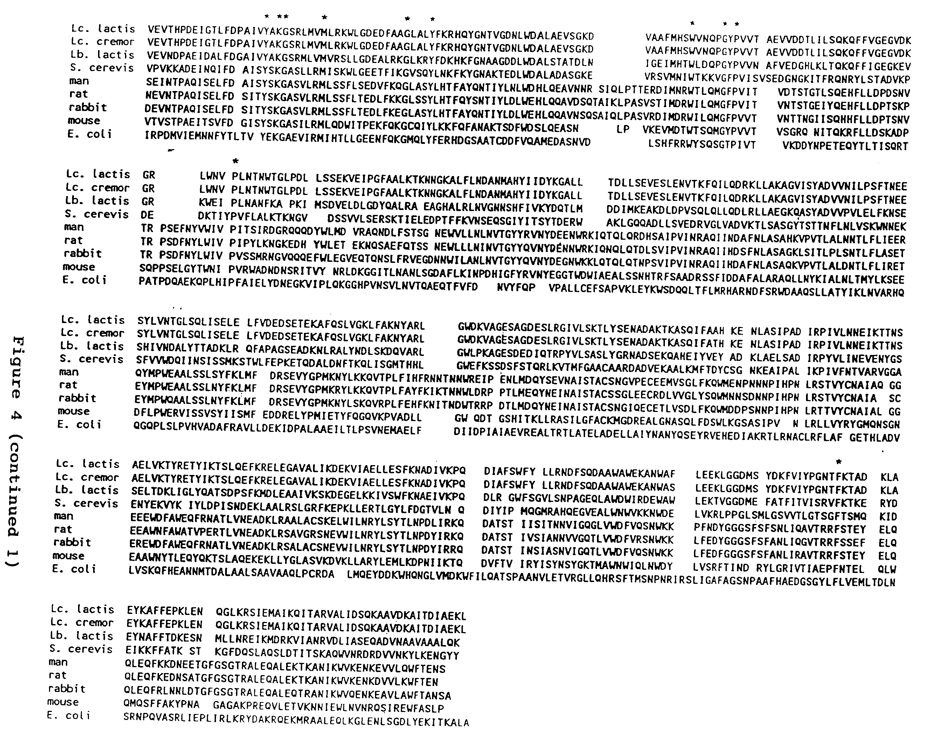

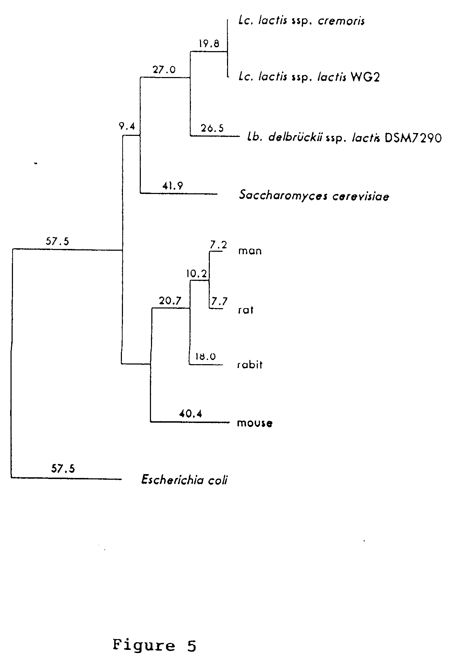

Searching the EMBL database resulted in similarities to several lys-aminopeptidases (FIG.4) belonging to the aminopeptidase N family (E.C. 3.4.11.2). These enzymes, sharing a common pattern of primary structure in the part of their sequence involved in the binding of zinc, can be grouped together as a family of neutral zinc metallopeptidases. They catalyse the removal of N-terminal amino acids from peptides. The most significant homology is observed in a functionally important segment which contains the proposed $Zn^{2+}$-binding catalytic site (Jongeneel et al., 1989). The phylogenetic tree calculated (FIG.5) demonstrates the close relationship of the lactobacillal enzyme to those of the lactococcal species. The diversity in the N-terminal sequences is a result from signal sequences present in human, rat, and mouse enzymes. The absence of a signal peptide sequence and the hydrophilicity plot of the amino acid sequence according to Kyte and Doolittle (1982) which does not show transmembrane domains, indicate that PepN of *Lb. delbrückii* ssp. *lactis* might be a intercellular located enzyme. Until now, no structure allowing protein secretion is described for any peptidase of lactic acid bacteria, but since the action of peptidases is required for casein cleavage a yet unknown mechanism might be responsible for translocation to the cell surface.

## Gene dosis effects

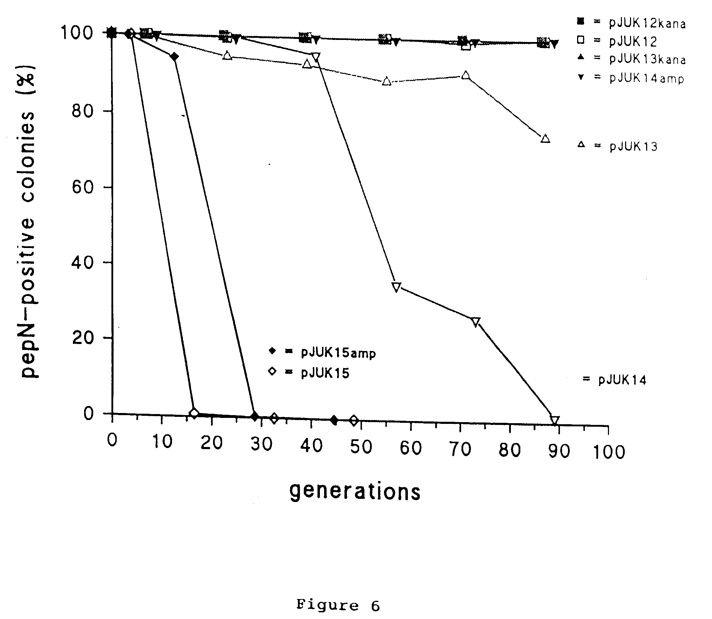

The knowledge of the *pepN* nucleotide sequence allowed subcloning of a minimal sized DNA fragment into different cloning vectors. As summarized in FIG.1 we succeeded in cloning a 2.9 kb DraI fragment of pJUK11 in both orientations into pLG339, thus indicating that the *pepN* promoter sequence is functional in *E. coli*. This fragment was also cloned into the medium copy number plasmid pBR322 and even into the high copy number plasmid pUC18. In the latter constructs only one of the two possible insert orientations were obtained, likely an effect of lethal overproduction, since *pepN* would then be under control of lac or tet promoters. The amount of PepN protein produced, increased with the copy number of the plasmids, but on costs of plasmid stability (FIG.6).

For homologous expression in a *Lactobacillus* starter culture the cloned peptidase gene was inserted in both orientations, as a *Sph*I-restriction fragment, into pJK355 (FIG.1), a *Lactobacillus* vector constructed from the cryptic plasmid from *Lb. curvatus* LTH683 (Klein et al., 1993) resulting in plasmids pJK361 and pJK362. In crude extracts of *Lb. casei* LK1, with a chromosomal *pepN* gene, a lys-pNA hydrolysing activity could be measured, but expression of *pepN* coded on plasmids pJK361 or pJK362 resulted in overproduction of enzyme visible in SDS-PAGE (FIG.3) and enzymatic activities in crude extracts were increased by 15 to 20 fold. This increase of PepN activity had no obvious effect on cell growth in MRS-broth, which could have been expected due to an imbalance in peptide supply. The influence on growth in milk is still going to be investigated as well as the effects on changes in taste and texture of cheese produced with such a modified strain. Purified PepN of *Lc. lactis* is reported to have a debittering activity on tryptic digests of β-casein (Tan et al., 1992). If the cloned and overexpressed enzyme of DSM7290 has comparable activities this gene can be of great industrial interest.

## Purification and partial characterization of the cloned peptidase N

Since PepN protein was extremely overexpressed in *E. coli* harbouring the plasmid coded gene, the enzyme could be purified to electrophoretic purity by use of a four-step procedure. Due to sequence homologies of the cloned lactobacillal *pepN* with the *Escherichia coli* aminopeptidase N gene, the enzymatic properties were investigated in CM89 a strain lacking the *E. coli* enzyme. CM89(pUK13) was chosen for purification because the plasmid was stable in the presence of Kanamycin and high yields of PepN protein were obtained. Table 2 summarizes the purification to electrophoretic purity by the use of streptomycin sulfate precipitation, anion exchange chromatographies on Q-sepharose, and gelfiltration on HW TSK 55(S). The final purification was approximately 11-fold with a recovery of 20 %. Estimated by SDS-PAGE and gelfiltration, the enzyme is a monomer with a molecular mass of 95 kDa, which agrees well with the value deduced from sequence analysis. The pI of 4.2, determined by isoelectric focusing agrees well with the predicted one of 4.48 deduced from amino acid sequence. Optimum PepN activity was measured at pH of 6.5 - 7.0 and temperatures of 45 - 55°C. Denaturation occurs at pH values below 4 and at temperatures above 60°C.

The classification based on the susceptibility to a group of protease inhibitors (PMSF, E-64, pepstatin, EDTA, and 1,10 phenanthroline) indicated that the enzyme belongs to the class of metallo proteases, as full inhibition with 1,10 phenanthroline or EDTA could be detected, whereas the inhibitors PMSF, Pepstatin A, or E-64 had no effect on enzyme activity. Additionally the homology to other aminopeptidases implies that PepN has a catalytic centre with zinc binding sites (FIG.4). The inhibition by both complexing agents could be specifically restored by addition of $Mn^{2+}$ and $Co^{2+}$, while $Zn^{2+}$ or $Mg^{2+}$ had no effect. Therefore one might suspect that $Mn^{2+}$ and $Co^{2+}$ ions play an essential role in the hydrolytic mechanism.

The substrate specificity of PepN against various p-nitroanilides is shown in Table 3. The highest activity was found for lys-p-nitroanilide and a Lineweaver-Burk plot indicated a Km of 70 μM. In comparison with the enzyme of *Lc. lactis* ssp. *cremoris* (Tan and Konings, 1990), showing the closest sequence relationship (62 % similarity) the lactobacillal Km value demonstrates an 8-fold higher affinity against lys-pNA. There are certain similar properties such as specifity against several p-NA substrates with bulk or aliphatic N-terminal residues, and pH optimum. The enzyme differs in restoration of enzyme activity after 1,10 phenanthroline inhibition. The temperature optimum of 50°C and its stability at elevated temperatures, after a 20 min incubation at 60°C a residual activity of 33 % were measured, might be of interest for applications of the cloned enzyme in milk fermentations.

## References

Brendel, V. & Trifonov, E.N. (1984) A computer algorithm for testing potential prokaryotic terminators. Nucleic Acids Res. 12, 4411-4427.

Chapot-Chartier, M.P., Nardi, M., Chopin, M.C., Chopin, A. & Gripon, J.C. (1993) Cloning of *pepC*, a cysteine aminopeptidase from *Lactococcus lactis* subsp. *cremoris* AM2. Appl. Environ. Microbiol. 59, 330-333

De Man, J.C., Rogosa, M. & Sharpe, M.E. (1960) A medium for the cultivation of lactobacilli. J. Appl. Bacteriol. 23, 130-135.

de Vos W.M. & Konings W.N. (1992) Characterization of the Lactococcus lactis pepN gene encoding an aminopeptidase homologous to mammamilan aminopeptidase N. FEBS lett. 306, 9-16

Dower, W.J., Miller, J.F. & Ragsdale, C.W. (1988) High efficiency transformation of E. coli by high voltage electroporation. Nucleic acids Res. 16, 2127-2145.

Feng, D.F. & Doolittle, R.F. (1987) Progressive sequence alignment as a prerequisite to correct phylogenetic trees. J. Mol. Evol. 25, 351-358.

Garcia-Alvarez, N., Cueva, R. & Suarez-Rendueles, P. (1991) Molecular cloning of soluble aminopeptidases from Saccharomyces cerevisiae: Sequence analysis of aminopeptidase yscII, a putative zinc-metallopeptidase. Eur. J. Biochem. 202, 993-1002.

Haandrikman, A.J., Kok, J., Laan H., Soemitro, S., Ledeboer, A.M., Konings, W.N. & Venema G. (1989) Identification of a gene required for maturation of an extracellular lactococcal serine proteinase. J. Bacteriol. 171, 2789-2794.

Henrich, B., Monnerjahn, M. & Plapp, R. (1990) Peptidase D gene (pepD) of Escherichia coli K-12: Nucleotide sequence, transcript mapping, and comparison with other peptidase genes. J. Bacteriol. 172, 4641-4651

Henrich, B. & Plapp, R. (1986) Use of the lysis gene product of bacteriophage iX174 for the construction of a positive selection vector. Gene. 42, 345-349.

Holck, A. & Naes, H. (1992) Cloning, sequencing and expression of the gene encoding the cell-envelope-associated proteinase from Lactobacillus paracasei subsp. paracasei NCDO 151. J. General Microbiol. 138, 1353-1364.

Jongeneel, C.V., Bouvier, J. & Bairoch, A. (1989) A unique signature identifies a family of zinc-dependent metallopeptidases. FEBS Lett. 242, 2111-214.

Kiwaki, M., Ikema, H., Shimizu-Kadota, M., & Hirashima A. (1989) Molecular characterization of a cell wall associated proteinase gene of Streptococcus lactis NCDO763. Mol. Microbiol. 3, 359-369.

Klein, J.R., Ulrich, C. & Plapp, R. (1993) Characterization and sequence analysis of a small cryptic plasmid from Lactobacillus curvatus LTH683 and its use for construction of new Lactobacillus cloning vectors. Plasmid, in press.

Kok, J., Leenhouts, . Handrikman, A.J., de Reuver, M.J.B., Laan H., & Venema, G. (1988) Nucleotide sequence of the cell wall proteinase gene of Streptococcus cremoris Wg2. Appl. environ. Microbiol. 54, 231-238.

Kyte, J. & Doolittle, R.F. (1982) A simple method for displaying the hydrophatic character of a protein. J. Mol. Biol. 157, 105-132.

Laemmli, U.K. (1970) Cleavage of structural proteins during the assembly of the head of bacteriophage T4. Nature 227, 680-685.

Law, J., Vos, P., Hayes, F., Daly, C., de Vos, W.M. & Fitzgerald, G. (1992) Cloning and partial sequencing of the proteinase gene complex from Lactococcus lactis subsp. lactis UC317. J. General Microbiol.Apr, 138, 709-18.

Lowry, O.H., Rosebrough, N.J., Farr A.L. & Randell, R.J. (1951) Protein measurement with the folin phenol reagent. J. Biol. Chem. 193, 265-275.

Malfroy, B., Kado-Fong H., Gros H.C., Giros B., Schwartz J.C. & Hellmiss, R. (1989) Molecular cloning and amino acid sequence of rat kidney aminopeptidase n: a member of a super family of zinc-metallohydrolases. Biochem. Biophys. Res. Commun. 161, 236-241

Mayo, B., Kok, K., Venema, K., Bockelmann, W., Teuber, M., Reinke, H. & Venema, G. (1991) Molecular cloning and sequence analysis of the X-prolyl-dipeptidyl-aminopeptidases gene from Lactococcus lactis subsp. cremoris. Appl. Environ. Microbiol. 57, 38-44.

McCaman, M.T. & Gabe, J.D. (1986) The nucleotide sequence of the pepN gene and its overexpression in Escherichia coli. Gene 48, 145-153

McCaman, M.T. & Gabe J.D. (1986) Sequence of the promoter and 5' coding region of pepN, and the amino-terminus of peptidase N from Escherichia coli K-12. Mol. Gen. Genet. 204, 148-152

Meyer-Barton, E.C., Klein, J.R., Imam, M. & Plapp, R. (1993) Cloning and sequence analysis of the X-prolyl-dipeptidyl-aminopeptidase gene (pepX) from Lactobacillus delbrückii ssp. lactis DSM 7290. Applied Microbiology and Biotechnology, 40, 82-89.

Miller, C.G., Mackinon, K. (1974) Peptidase mutants of Salmonella typhimurium. J. Bacteriol. 120, 355-363.

Miller, C.G. & Schwartz, G. (1978) Peptidase deficient mutants of Escherichia coli. J. Bacteriol. 135, 603-611.

Miller, J.H. (1972) Experiments in Molecular Genetics. Cold Spring Harbor Laboratory, Cold Spring Harbor, New York.

Nardi, M., Chopin, M., Chopin, A., Cals, M. & Gripon, J. (1991) Cloning and DNA sequence analysis of an X-prolyl dipeptidyl aminopeptidase gene from Lactococcus lactis subsp. lactis NCDO 763. Appl. Environ. Microbiol. 57, 45-50.

Noren, O., Dabelsteen, E., Hoyer, P.E., Olsen, J., Sjoestroem, H. & Hansen, G.H. (1989) Onset of transcription of the aminopeptidase N (leukemia antigen CD 13) gene at the crypt/villus transition zone during rabbit enterocyte differentiation. FEBS Lett. 259, 107-112.

Olsen, J., Cowell, G.M., Koenigshoefer, E., Danielsen, E.M., Moeller, J., Laustsen, L., Hansen, O.C., Welinder, K.G., Engberg, J., Hunziker, J.W., Spiess, M., Sjoestroem, M.H. & Noren, O. (1988) Complete amino acid sequence of human intestinal aminopeptidase N as deduced from cloned cDNA. FEBS Lett. 238, 307-314.

Olsen, J., Sjoestroem, H. & Noren, O. (1989) Cloning of the pig aminopeptidase N gene. FEBS Lett. 251, 275-281.

Raleigh, E.A., Murray, N.E., Revel, H., Blumenthal, R.M., Westawy, D., Reith, A.D., Rigby, P.W.J., Elhai, J. & Hanahan, D. (1988) McrA and McrB restriction phenotypes of some E. coli strains and implication for gene cloning. Nucl. Acids Res. 16, 1563-1575

Sambrook, J., Fritsch, E.F. & Maniatis, T. (1989) Molecular cloning: a loboratory manual, 2nd edn. Cold Spring Harbor Laboratory, Cold Spring Harbor, NY.

Stoker, N.G., Faiweather, N.F. & Spratt, B.G. (1982) Versatile low copy number plasmid vectors for cloning in *Escherichia coli*. Gene 18, 335-341.

Stroman, P. (1992) Sequence of a gene (lap) encoding a 95.3-kDA aminopeptidase from *Lactococcus lactis* ssp. *cremoris* Wg2. Gene 113, 107-112

Sanger, F., Nicklen, S. & Coulson, A.R, (1977) DNA sequencing with chain-terminating inhibitors. Proc. Natl. Acad. Sci. USA 74, 5463-5467.

Sutcliffe, J.G. (1979) Complete nucleotide sequence of the *E. coli* plasmid pBR322. Cold Spring Harbor Symp. Quant. Biol. 43, 77-90.

Tan, P.S.T. & Konings, W.N. (1990) Purification and characterization of an aminopeptidase from *Lactococcus lactis* ssp. *cremoris* Wg2. Appl. Environm. Microbiol. 56, 526-532.

Tan, P.S.T., van Kessel, A.J.M., van de Veerdonk, F.L.M., Zuurendonk, P.F., Bruins A.P. & Konings W.N. (1992) Degradation and debittering of a tryptic digest from β-casein by aminopeptidase N from *Lactococcus lactis* subsp. *cremoris* WG2. Thesis.

Tan, P.S.T., van Alen-Boerrigter, I.J., Poolman, B., Siezen R.J., de Vos, W.M. & Konings, W.N. (1992) Characterization of the *Lactococcus lactis pepN* gene encoding an aminopeptidase homologous to mammalian aminopeptidase N. FEBS lett. 306, 9-16.

Van Alen-Boerrigter, I.J., Baankreis, R. & de Vos, W.M. (1991) Caracterization and overexpression of the *Lactococcus lactis pepN* gene and localization of its product, aminopeptidase N. Appl. Environ. Microbiol. 57, 2555-2561.

Van de Guchte, M., Kok, J. & Venema, G. (1992) Gene expression in *Lactococcus lactis*. FEMS Microbiol. Rev., 88, 73-92.

Vos, P., Simons, G., Siezen, R.J., & de Vos W.M., (1989a) Primary structure and organization of the gene for a procaryotic cell-envelope-located serine proteinase. J. Biol. Chem. 264, 13579-13588.

Vos, P., van Asseldonk, M., van Jeveren, F., Siezen, R., Simons, G. & de Vos, W.M. (1989b) A maturation protein is essential for production of active forms of *Lactococcus lactis* SK11 serine proteinase located in or secreted from cell envelope. J. Bacteriol. 171, 2795-2802.

Wu, Q., Lahti, J.M., Air, G.M., Burrows, P.D. & Cooper, P.D.M.D. (1990) Molecular cloning of the murine BP-1/6C3 antigen: A member of the zinc-dependent metallopeptidase family. Proc. Natl. Acad. Sci. U.S.A. 87, 993-997.

Yanisch-Peron, C., Vieira, J. & Messing, J. (1985) Improved M13 phage cloning vectors and host strains: nucleotide sequence of the M13mp18 and pUC19 vectors. Gene 33, 103-119.

Zink, A., Klein, J.R. & Plapp, R. (1991) Transformation of *Lactobacillus delbrückii* ssp. *lactis* by electroporation and cloning of origins of replication by use of a positive selection vector. FEMS Microbiol. Letters 78, 207-212.

## Legends to the figures

FIG. 1. Circular map of plasmid pJUK11. The insert of chromosomal DNA of 5.2 kbp in size, is indicated as a shaded segment, with the *pepN* gene presented as dotted arrow. The linear expansion of the insert illustrates the sequencing strategy. The different restriction fragments used for subcloning into different vectors, all spanning the *pepN* gene, are indicated as black bars.

FIG. 2. Expression of PepN protein in *E. coli* (ER1562) and *Lb. casei* (LK1). Separation of crude cell extracts by SDS-PAGE (12 % acrylamide). Proteins were visualized by Coomassie staining. Lanes 1 and 2, molecular weight marker proteins; cell extracts of *E. coli* ER1562(pJUK11) [lane3] and ER1562(pLG339) [lane4]; cell extracts of LK1 [lane 7], LK1(pJK361) [lane5] and LK1(pJK362) [lane 6]. The position of PepN is marked by an arrow head.

FIG. 3. Nucleotide sequence of the *pepN* region and deduced amino acid sequence of PepN. The proposed -35 (212 to 234) and -10 (250 to 255) positions and ribosome binding site (305 to 309) are underlined. An inverted repeat (underlined at bps 2871 to 2891) with a dG value of -12.8 kcal/mol, followed by a run of T-residues, is proposed as a transcription terminator.

FIG. 4. Progressive alignment (TREE, software of HUSAR GENIUSnet, Heidelberg) of the protein sequence of PepN from *Lb. delbrückii* ssp. *lactis* DSM7290 with those from other aminopeptidases. Identical amino acids in the sequences are marked (*). The putative catalytic sites (A) with zinc-binding domains (Z) are indicated.

FIG. 5. Phylogenetic relationship of aminopeptidases from different species. The dendrogram was calculated from the progressive alignment of homologous protein sequences using the program TREE of HUSAR, which is based on the method described by Feng and Doolittle (1987). The vertical bars represent nucleotide changes in percent.

FIG. 6. Stability of different plasmids coding for *pepN* in *Escherichia coli*. ER1562 harbouring different plasmids (pJUK12, pJUK13, pJUK14, pJUK15) was grown in liquid medium with and without the appropriate antibiotic selection.

EP 0 633 316 A1

Table 1. Bacterial strains and plasmids

| Strain | | relevant genotype or phenotype | Reference or source |
|---|---|---|---|
| Lactobacillus casei | LK1 [a] | | Zink et al., 1991 |
| Lactobacillus delbrückii ssp. lactis | DSM7290 [b] | source of library, pepN[+] | M. Busse [c] |
| Escherichia coli K-12 | ER1562 | EcoK r[-]m[+], mcrA[-] mcrB[-] | Raleigh et al. 1988, Biolabs |
| Escherichia coli K-12 | CM89 | pepN[-] | Miller and Schwartz, 1978 |

| Plasmid | replicon, relevant feature | Reference or source |
|---|---|---|
| pUC18 | ColE1, high copy number | Yanish-Peron et al., 1985 |
| pUM84 | ColE1, high copy number, positive selection for insert DNA | Henrich and Plapp, 1986 |
| pBR322 | ColE1, medium copy number | Sutcliffe, 1979 |
| pLG339 | pSC101, low copy number | Stoker et al. 1982 |
| pJK355 | pLC2, cryptic plasmid from Lb. curvatus LTH683 | Klein et al. 1993 |
| pJUK11 | pSC101 from pLG339 | this work |
| pJUK12 | pSC101 from pLG339 | this work |
| pJUK13 | pSC101 from pLG339 | this work |
| pJUK14 | ColE1 from pBR322 | this work |
| pJUK15 | ColE1 from pUC18 | this work |
| pJK360 | pLC2 from pJK355 | this work |
| pJK361 | pLC2 from pJK356 | this work |

[a] formerly referred to as WS97

[b] formerly referred to as WS87

[c] Weihenstephan, F.R.G.

Table 2. Purification of aminopeptidase N from *Lb. delbrückii* spp. *lactis* DSM7290

| Purification step | Total activity[a] $(U \times 10^6)$ | Specific activity[b] | Yield (%) | Purification (fold) |
|---|---|---|---|---|
| Cell extract | 2 | 4170 | 100 | 1 |
| Streptomycin sulfate precipitation | 1.96 | 4990 | 98 | 1.2 |
| Q-Sepharose (0-0.5 M NaCl) | 1.6 | 11350 | 80 | 2.7 |
| Q-Sepahrose (0.4-0.7 M NaCl) | 1.1 | .22920 | 55 | 5.5 |
| TSK HW 55(S) | 0.4 | 45980 | 20 | 11.0 |

[a] 1 Unit defined as realease of 1 μmole p-nitroaniline per minute at 37°C
[b] specific activity defined as units per mg of protein

Table 3. Relative activity of PepN for p-nitroanilide substrates

| p-nitroanilide (-pNA) substrate | relative activity (%) |
|---|---|
| Lys-pNA | 100 |
| Leu-pNA | 19 |
| Ala-pNA | 12 |
| Phe-pNA | 8 |
| Pro-pNA | 2 |
| Tyr-pNA | 2 |
| Gly-pNA | 1 |
| Ala-Pro-pNA | 0 |

**Claims**

1.  Protein in a substantially pure form, as produced and excreted by *Lactobacillus delbrückii* ssp. *lactis* and capable of hydrolysing aminoacid β-naphtylamides, and more particularly lysine-β-naphtylamide, or fragments thereof having this enzymatic activity.

2.  Protein according to claim 1, such as produced by *Lactobacillus delbrückii* ssp. *lactis* WS87, deposited at the Deutsche Sammlung von Mikroorganismen (DSM) under the number 7290 on October 15, 1992.

3.  Protein according to claim 1 or claim 2, characterized in that it corresponds to the one (also called PepN) represented on figure 3, and by SEQ ID NO 2, or fragments or muteins (which differ from said protein by addition and/or substitution and/or suppression of one or several amino acid) thereof, provided that said fragments and muteins are capable of hydrolysing β-naphtylamides.

4.  Protein according to anyone of claims 1 to 3, characterized in that:
    -   its isoelectric point calculated from the nucleotide sequence represented by SEQ ID NO 1, is 4,48, and its isoelectric point determined by preparative isoelectric focusing after purification of said protein, is 4,2,
    -   its molecular weight calculated from the nucleotide sequence represented by SEQ ID NO 1, is 95,358 kDa, and its molecular weight determined after purification, is 95 kDa,
    -   its specific inhibitors are the following:
        .   EDTA: end concentration 1mM; 3 % relative activity,
        .   phenanthroline: end concentration 1 mM: 4 % relative activity.

5.  Protein according to anyone of claims 1 to 4, characterized in that it is obtained in a purified state, from a cell extract of said *Lactobacillus delbrückii* ssp. *lactis* WS87 by the following procedure:
    -   fractionation of the cell extract by salting out at 4°C with streptomycin sulfate,
    -   centrifugation,
    -   anion exchange chromatography by applying the supernatant obtained at the previous step to an appropriate column, such as a column of Q-Sepharose Fast Flow (Pharmacia), and pooling the eluted fractions having the highest specific activity against L-Pro-p-nitroanilide,
    -   preparative isoelectric focusing, by applying the pooled fractions obtained at the previous step to an appropriate column, such as a LKB column (Types 8100-1), with ampholytes within a range of pH 4 to 6, and pooling the eluted fractions having the highest specific activity against Lys-paranitroanilide.

6.  Nucleic acid characterized in that it codes for a protein according to anyone of claims 1 to 5.

7.  Nucleic acid according to claim 6, characterized in that:
    -   it comprises all or part of the nucleic acid represented on figure 3, and by SEQ ID NO 1, coding for a protein according to anyone of claims 1 to 5, or its complementary sequence,
    -   or it hybridizes with all or part of said nucleic acid represented by SEQ ID NO 1, or with its complementary sequence.

8.  Nucleic acid according to claim 6 or claim 7, characterized in that it codes for the PepN protein represented by SEQ ID NO 2, or for polypeptides derived thereof having said hydrolysing β-naphtylamides activity, said nucleic acid comprising all or part of the nucleotide sequence delimited by the nucleotide located in position 316 and the nucleotide located in position 2844 in the nucleotide sequence represented by SEQ ID NO 1.

9.  Recombinant nucleic acid containing at least one nucleic acid according to anyone of claims 6 to 8, inserted into a heterologous nucleic acid.

10. Recombinant vector comprising a vector sequence, notably of the type plasmid (such as plasmids originating from lactic acid bacteria), cosmid or phage, and a nucleic acid according to anyone of claims 6 to 8, in one of the non-essential sites for its replication, and optionally one or several nucleic acid(s) coding for other aminopeptidases such as PepX.

11. Cellular host which is transformed by a recombinant vector according to claim 10, and comprising the regulation elements enabling the expression of the nucleotide sequence coding for a protein according to anyone of claims 1 to 5, and optionally, other aminopeptidases such as PepX, in this host.

12. Cellular host according to claim 12, chosen from among bacteria such as *E.coli*, or lactic acid bacteria including *lactococcus* and, especially, lactobacilli of the thermophilic group, transformed by a vector according to the invention.

13. Process for preparing a protein according to anyone of claims 1 to 5 comprising the following steps:
    - the culture in an appropriate medium of a cellular host according to claim 11 or 12,
    - the recovery of the polypeptide produced by the above said cellular host from the above said culture medium,
    - the purification of the protein thus obtained.

14. Fermentation process characterized in that it comprises a step of treatment of material to be fermented with:
    - an appropriate amount of the PepN protein or polypeptides derived thereof according to claims 1 to 5, and optionally other proteases, and more particularly aminopeptidases such as PepX, and/or
    - an appropriate amount of at least one of the transformed cellular host, according to claim 11 or 12,
    - and optionally, an appropriate amount of lactobacilli, such as *Lactobacillus delbrückii* ssp. *lactis*.

15. Process for the preparation of fermented foodstuff, and more particularly of cheese, which comprises a step of treatment of food material to be fermented, such as milk, with:
    - an appropriate amount of at least one of the protein according to claims 1 to 5, and optionally other aminopeptidases such as PepX, and/or
    - an appropriate amount of at least one of the transformed cellular host according to of claim 11 or 12, and/or
    - an appropriate amount of lactobacilli, such as *Lactobacillus delbrückii* ssp. *lactis*.

16. Process according to claim 15, characterized in that it comprises a step of treatment of food material to be fermented, such as milk, with other species and strains susceptible to be used as starter organisms in fermentation processes, and more particularly lactic acid bacteria susceptible to produce a PepX protein, such as *Lactococcus*, *Streptococcus* and *Lactobacillus*.

17. Process according to claim 15 or claim 16, characterized in that it can be used as fermentation processes for the obtention of hard cheeses, such as Emmentaler type cheese

18. Foodstuff, and more particularly cheeses, such as obtained by fermentation processes, according to anyone of claims 15 to 17.

Figure 1

Figure 2

```
          10        20        30        40        50        60
GATAGGGCTGAAATTATCATTTTAAGCGCTTTAAATTAGCTATACAGATAAGTAACATTA

          70        80        90       100       110       120
GTAACAATTGTCAAGAGACTGCAATAAAAGGAAAAGGCCAGCTGCTAGACTGGTCTTTTA

         130       140       150       160       170       180
CATATGCAATTATTTCAAAAATGGAATTAATTTCCGTTAGAAACTGAATTGTCTGATCGA

         190       200       210       220       230       240
GTTCAAGGGGCTGAGGTAGACTGCAAACAGATATTTTGCGTTAAATGGGCTTTATTTAGC
                                              -35        proposed

         250       260       270       280       290       300
CTTTTTTGCTAGAATAGAGAAGTGTGAATACAATATACGCGAGGAAAAATCAGACGCGGA
promoter    -10

translation initiation        330       340       350       360
TTATAGGAGGAAACAATGGCTGTTAAGCGTTTTTACGAAACATTCCATCCAGATCACTAC
                   MetAlaValLysArgPheTyrGluThrPheHisProAspHisTyr

         370       380       390       400       410       420
GATCTATACATCGACGTTGACCGGGCAGCAAGATCTTTCTCAGGGACTTCCACCATCCAT
AspLeuTyrIleAspValAspArgAlaAlaArgSerPheSerGlyThrSerThrIleHis

         430       440       450       460       470       480
GGTGAAATCCAGGAAGAAACTGTCTTGGTCCACCAAAAGTACATGACCATCAGCAAGGTC
GlyGluIleGlnGluGluThrValLeuValHisGlnLysTyrMetThrIleSerLysVal

         490       500       510       520       530       540
ACTGTTGACGGCAAGGAAGTGCCGTTTACTTTTGGCGACGACTTTGAAGGCATCAAGATC
ThrValAspGlyLysGluValProPheThrPheGlyAspAspPheGluGlyIleLysIle

         550       560       570       580       590       600
GAAGCCGGCAAGACTGGGGAAGCGGTCATCGCGATCGACTACTCAGCGCCTTTGACTGAC
GluAlaGlyLysThrGlyGluAlaValIleAlaIleAspTyrSerAlaProLeuThrAsp
```

**Figure 3**

29

```
         610       620       630       640       650       660
ACTATGATGGGGATCTACCCGTCCTACTACCAAGTTGATGGCGTCAAGAAGGAACTGATC
ThrMetMetGlyIleTyrProSerTyrTyrGlnValAspGlyValLysLysGluLeuIle

         670       680       690       700       710       720
GGGACCCAGTTTGAAACTACTTTTGCCCGGGAAGCCTTCCCATGTGTTGACGAACCAGAA
GlyThrGlnPheGluThrThrPheAlaArgGluAlaPheProCysValAspGluProGlu

         730       740       750       760       770       780
GCTAAGGCAACCTTCTCCCTCGCCCTCAAGTTTGACGAACACGAAGGCGAAACTGTTTTG
AlaLysAlaThrPheSerLeuAlaLeuLysPheAspGluHisGluGlyGluThrValLeu

         790       800       810       820       830       840
GCCAACATGCCAGAAGACCGGGTAGAAAACGGCGTGCACTACTTTAAGGAGACTGTCCGC
AlaAsnMetProGluAspArgValGluAsnGlyValHisTyrPheLysGluThrValArg

         850       860       870       880       890       900
ATGTCCAGCTACCTGGTTGCTTTTGCCTTTGGTGAAATGCGGTCATTGACCACCCACACC
MetSerSerTyrLeuValAlaPheAlaPheGlyGluMetArgSerLeuThrThrHisThr

         910       920       930       940       950       960
AAGAGCGGGGTCTTGATCGGGGTTTACTCAACTCAAGCCCACACTGAAAAGGAACTGACC
LysSerGlyValLeuIleGlyValTyrSerThrGlnAlaHisThrGluLysGluLeuThr

         970       980       990      1000      1010      1020
TTCTCCTTGGACATTGCCAAGCGGGCAATTGAATTCTACGAAGACTTTTACCAAACTCCA
PheSerLeuAspIleAlaLysArgAlaIleGluPheTyrGluAspPheTyrGlnThrPro

        1030      1040      1050      1060      1070      1080
TATCCGCTGCCGCAATCACTGCAGCTGGCCCTGCCTGACTTCTCAGCCGGTGCCATGGAA
TyrProLeuProGlnSerLeuGlnLeuAlaLeuProAspPheSerAlaGlyAlaMetGlu

        1090      1100      1110      1120      1130      1140
AACTGGGGTCTGGTAACCTACCGGGAAGCCTACCTGCTTTTGGACCCGGACAACACCACT
AsnTrpGlyLeuValThrTyrArgGluAlaTyrLeuLeuLeuAspProAspAsnThrThr
```

```
        1150      1160      neutral Zn metallopeptidase signature
TTGGAAATGAAGAAGCTGGTTGCGACTGTGGTGACCCACGAACTGGCCCACCAATGGTTC
LeuGluMetLysLysLeuValAlaThrValValThrHisGluLeuAlaHisGlnTrpPhe
                                  Zn Act        Zn
```

Figure 3 (continued 1)

30

```
        1210      1220      1230      1240      1250      1260
GGTGACCTGGTAACCATGGAATGGTGGGACAACCTCTGGCTGAACGAAAGTTTCGCCAAC
GlyAspLeuValThrMetGluTrpTrpAspAsnLeuTrpLeuAsnGluSerPheAlaAsn


        1270      1280      1290      1300      1310      1320
ATGATGGAATACCTGTCAGTTGACCACCTGGAACCTAACTGGCACATCTGGGAAATGTTC
MetMetGluTyrLeuSerValAspHisLeuGluProAsnTrpHisIleTrpGluMetPhe


        1330      1340      1350      1360      1370      1380
CAGACTTCTGAAGCAGCGGCTGCCTTGACCCGGGATGCAACCGACGGGGTACAGTCAGTG
GlnThrSerGluAlaAlaAlaAlaLeuThrArgAspAlaThrAspGlyValGlnSerVal


        1390      1400      1410      1420      1430      1440
CACGTGGAAGTTAATGACCCGGCTGAAATCGACGCCCTCTTTGACGGGGCCATCGTTTAC
HisValGluValAsnAspProAlaGluIleAspAlaLeuPheAspGlyAlaIleValTyr


        1450      1460      1470      1480      1490      1500
GCCAAGGGGTCAAGAATGCTGGTCATGGTCCGGTCACTTTTGGGCGATGAAGCCTTGAGA
AlaLysGlySerArgMetLeuValMetValArgSerLeuLeuGlyAspGluAlaLeuArg


        1510      1520       1530     1540      1550      1560
AAGGGCTTGAAGCGCTACTTTGACAAGCACAAGTTTGGCAACGCGGCAGGTGACGATCTC
LysGlyLeuLysArgTyrPheAspLysHisLysPheGlyAsnAlaAlaGlyAspAspLeu


        1570      1580      1590      1600      1610      1620
TGGGATGCCTTGTCAACGGCCACTGACTTGAACATTGGGGAAATCATGCACACTTGGCTG
TrpAspAlaLeuSerThrAlaThrAspLeuAsnIleGlyGluIleMetHisThrTrpLeu


        1630      1640      1650      1660      1670      1680
GACCAGCCAGGCTACCCAGTGGTGAATGCTTTTGTTGAGGACGGCCACTTGAAGCTGACT
AspGlnProGlyTyrProValValAsnAlaPheValGluAspGlyHisLeuLysLeuThr


        1690      1700      1710      1720      1730      1740
CAGAAGCAATTCTTCATCGGTGAAGGCAAGGAAGTCGGCCGCAAGTGGGAAATTCCGCTT
GlnLysGlnPhePheIleGlyGluGlyLysGluValGlyArgLysTrpGluIleProLeu


        1750      1760       1770     1780      1790      1800
AACGCTAACTTCAAGGCACCGAAGATCATGTCAGACGTTGAACTTGACCTGGGTGACTAC
AsnAlaAsnPheLysAlaProLysIleMetSerAspValGluLeuAspLeuGlyAspTyr
```

Figure 3 (continued 2)

```
        1810        1820        1830        1840        1850        1860
CAGGCTCTGCGGGCAGAAGCCGGCCACGCTCTGCGCTTGAACGTGGGCAACAACTCCCAC
GlnAlaLeuArgAlaGluAlaGlyHisAlaLeuArgLeuAsnValGlyAsnAsnSerHis


        1870        1880        1890        1900        1910        1920
TTCATCGTGAAGTACGACCAGACTTTGATGGACGACATCATGAAGGAAGCCAAGGACTTG
PheIleValLysTyrAspGlnThrLeuMetAspAspIleMetLysGluAlaLysAspLeu


        1930        1940        1950        1960        1970        1980
GATCCAGTTTCCCAATTGCAATTGCTGCAAGACCTGCGGCTTTTGGCAGAAGGCAAGCAG
AspProValSerGlnLeuGlnLeuLeuGlnAspLeuArgLeuLeuAlaGluGlyLysGln


        1990        2000        2010        2020        2030        2040
GCTTCATACGCTGACGTGGTACCAGTTCTGGAACTCTTCAAGAACTCAGAAAGCCACATT
AlaSerTyrAlaAspValValProValLeuGluLeuPheLysAsnSerGluSerHisIle


        2050        2060        2070        2080        2090        2100
GTCAACGATGCTCTGTACACGACTGCTGATAAGCTGCGGCAATTTGCCCCAGCCGGCAGT
ValAsnAspAlaLeuTyrThrThrAlaAspLysLeuArgGlnPheAlaProAlaGlySer


        2110        2120        2130        2140        2150        2160
GAAGCTGACAAGAACCTGCGGGCTCTGTACAACGACTTGTCCAAGGACCAAGTTGCCCGT
GluAlaAspLysAsnLeuArgAlaLeuTyrAsnAspLeuSerLysAspGlnValAlaArg


        2170        2180        2190        2200        2210        2220
TTGGGCTGGCTGCCTAAGGCAGGGGAAAGCGATGAAGACATTCAGACCCGGCCATACGTT
LeuGlyTrpLeuProLysAlaGlyGluSerAspGluAspIleGlnThrArgProTyrVal


        2230        2240        2250        2260        2270        2280
TTGTCTGCTAGCCTTTACGGCCGCAACGCTGATTCAGAAAAGCAAGCCCACGAAATCTAC
LeuSerAlaSerLeuTyrGlyArgAsnAlaAspSerGluLysGlnAlaHisGluIleTyr


        2290        2300        2310        2320        2330        2340
GTGGAATACGCTGATAAGTTGGCAGAACTGTCCGCTGATATCCGGCCATACGTTTTGATC
ValGluTyrAlaAspLysLeuAlaGluLeuSerAlaAspIleArgProTyrValLeuIle


        2350        2360        2370        2380        2390        2400
AACGAAGTTGAAAACTACGGGTCAAGCGAATTGACTGACAAGCTGATTGGTTTGTACCAG
AsnGluValGluAsnTyrGlySerSerGluLeuThrAspLysLeuIleGlyLeuTyrGln
```

Figure 3 (continued 3)

32

```
      2410       2420       2430       2440       2450       2460
GCAACCAGTGACCCATCATTCAAGATGGACCTGGAAGCCGCGATCGTGAAGAGCAAGGAC
AlaThrSerAspProSerPheLysMetAspLeuGluAlaAlaIleValLysSerLysAsp


      2470       2480       2490       2500       2510       2520
GAAGGCGAACTGAAGAAGATCGTTTCCTGGTTCAAAAACGCTGAAATCGTTAAGCCGCAG
GluGlyGluLeuLysLysIleValSerTrpPheLysAsnAlaGluIleValLysProGln


      2530       2540       2550       2560       2570       2580
GACTTGCGCGGCTGGTTCAGCGGCGTTTTGTCCAACCCGGCAGGTGAACAGCTGGCCTGG
AspLeuArgGlyTrpPheSerGlyValLeuSerAsnProAlaGlyGluGlnLeuAlaTrp


      2590       2600       2610       2620       2630       2640
GACTGGATCAGAGACGAATGGGCATGGTTGGAAAAGACGGTCGGCGGCGACATGGAATTC
AspTrpIleArgAspGluTrpAlaTrpLeuGluLysThrValGlyGlyAspMetGluPhe


      2650       2660       2670       2680       2690       2700
GCTACCTTCATCACTGTCATCTCCCGCGTCTTCAAGACCAAGGAACGCTACGACGAATAC
AlaThrPheIleThrValIleSerArgValPheLysThrLysGluArgTyrAspGluTyr


      2710       2720       2730       2740       2750       2760
AACGCCTTCTTTACTGACAAGGAAAGCAACATGCTGCTGAACCGGGAAATCAAGATGGAC
AsnAlaPhePheThrAspLysGluSerAsnMetLeuLeuAsnArgGluIleLysMetAsp


      2770       2780       2790       2800       2810       2820
CGGAAGGTCATCGCTAACCGGGTAGACTTGATTGCCAGCGAACAAGCTGACGTCAACGCC
ArgLysValIleAlaAsnArgValAspLeuIleAlaSerGluGlnAlaAspValAsnAla


      2830       2840       2850            proposed transcription
GCGGTTGCTGCTGCTTTGCAAAAGTAATTGAATAGAGCATAAGAAAACTGTTTCCGCTGA
AlaValAlaAlaAlaLeuGlnLysEnd


      2890       2900       2910       2920       2930       2940
GAGCTGGAAACAGTTTTTTTATGTATTCAACTTGTCTGCAATCGGTTACAATATAGATGT
terminator


      2950       2960       2970       2980       2990       3000
AAATACTATCGTACATTCTTTGAGGTAATAAAATGAACAACGATTTTAAAGATATCATGC
```

Figure 3 (continued 4)

```
     3010        3020        3030        3040        3050        3060
AAAACAGAAAGTCTATCCGGCACTATGATTCCAGCGTGAAGATTTCCCGTGACGAATTGC


     3070        3080        3090        3100        3110        3120
TGGAAATCATTAATGAATCTATCTCTGCTCCAAGTGCCTGCAACCTGCAGTCCTGGAAGC


TT
```

Figure 3 (continued 5)

| EMBL entry | ORGANISM | REFERENCE | HOMOLOGY in complete protein sequence (conserved amino acid changes permitted) | Abreviation in alignment |
|---|---|---|---|---|
| LLPEPN | Lc. lactis ssp. lactis MG1363 | Tan et. al. 1992a | 62 % | Lc. lactis |
| LLLYSAP | Lc. lactis ssp. cremoris Wg2 | Strøman 1992 | 62 % | Lc. cremor |
| OCAMINO | rabbit | Noren et al. 1989 | 41 % | rabbit |
| HSAMPEPN | Homo sapiens | Olsen et al. 1988 | 45 % | man |
| M26710 | rat | Helmiss 1989 | 44 % | rat |
| MMANTBP1 | mouse | Wu et al. 1990 | 44 % | mouse |
| SCAPE2G | Saccharomyces cerevisiae | Garcia-Alvarez et al. 1991 | 47 % | S. cerevis |
| ECPEPN | Escherichia coli | McCaman and Gabe 1986 | 39 % | E. coli |

```
Lc. lactis                                                                          MAVKR      LIETFVPENYKIF LDIDRKTKK   IKGQVAIT
Lc. cremor                                                                          MAVKR      LIETFVPENYKIF LDIDRKTKK   IKGQVAIT
Lb. lactis                                                                          MAVKR      FYETFHPDHYDLY IDVDRAARS   FSGTSTIH
S. cerevis                                                                          MTSKTPNREILPDNVVPLHYDLT VEPDFKTFK   FEGSVKIE
man             MAKGFYI SKSLGIL GILLGVAAVCTIIALSVVYSQEKNKNANSSPVASTTPSASATTNPASATTLDQSKAWNRYRLPNTLKPDSYQVT LRPYLTPNDRGLYVFKGSSTVR
rat             MAKGFYI SKTLGIL GILLGVAAVCTIIALSVVYAQEKNRNAENSAIAPTLPGSTSATTSTTNPAIDESKPWNQYRLPKTLIPDSYQVT LRPYLTPNEQGLYIFKGSSTVR
rabbit
mouse           MNFAEEEPSKKYCIKGKHVAIICGVVVAVGLIVGLSVGLTRSCEQDTTPAPSQPPPEASTALPPQDQNVCPDSEDESGEWKNFRLPDFINPVHYDLE VKALMEEDR   YTGIVTIS
E. coli                                                                             MTQQP  QAKYRHDYRAPDYQITDIDLTFDLDAQKTVVTAVSQAVR

Lc. lactis GEAKD   TVVSFHTKGLHFNKVRAFSVDTNFIENEEDEEIV       VKIGETGRVT       VSFEYEAELTDNMHGIYPSYYE  VNGEKKMLIGT  QFESHFARQAFP
Lc. cremor GEAKD   TVVAFHAKGLHFNKVRAFSVDTNFIENEEDEEIV       VKIGETGRVT       VSFEYEAELTDNMHGIYPSYYE  VNGEKKMLIGT  QFESHFARQAFP
Lb. lactis GEIQE   ETVLVHQKYMTISKVTVDGKEVPFTFGDDFEGIK       IEAGKTGEAV       IAIDYSAPLTDTMMGIYPSYYQ  VDGVKKELIGT  QFETTFAREAFP
S. cerevis LKINNPAIDTVTLNTVDTDIHSAKIGDVTSSEIISEEEQQVTT   FAFPKGTMSSFKGNAF     LDIKFTGILNDNMAGFYRAKYEDKLTGETKYMATT  QMEPTDARRAFP
man        FTCKEAT DVIIIHSKKLNYTLSQGHRVVLRGVGGSQPPDIDKTELVEPTEYLVVHLKGSLVK  DSQYEMDSEFEGELADDLAGFYRSEYM  EGNVRKVVATT  QMQAADARKSFP
rat        FTCNETT NVIIIHSKKLNYTNKGNHRVALRALGDTPAPNIDTTELVERTEYLVVHLQGSLVK  GHQYEMDSEFQGELADDLAGFYRSEYM  EGGNKKVVATT  QMQAADARKSFP
rabbit                                                                      QFQGELADDLAGFYRSEYM  EGNVRKVVATTQMQMQAADARKSFP
mouse      VNLSKPT RDLWLHIRETKITKLPE    LRRPSGEQVP  IRRCFEYKKQEYVVIQAAEDLAATSGDSVYRLTMEFKGWLNGSLVGFYKTTYM  EDGQIRSIAAT  DHEPTDARKSFP
E. coli    HGASD   APLRLNGEDLKLVSVHINDEPWTAWKEEEGALVI        SNLPERFTLK       IINEISPAANTALEGLYQS         GDALCT   QCEAEGFRHITY

Lc. lactis SIDEPEAKATFDLSVKFDE EEGDIIVSNMPE    LLNING  IHVFERTVKMSSYLLAFVFGELQYKKG  KTKSGVEVGAFATKAH SQAALDFPLDIAIRSIEFYEDYYQTPYPLP
Lc. cremor SIDEPEAKATFDLSVKFDE EEGDIIVSNMPE    LLNING  IHVFERTVKMSSYLLAFVFGELQYKKG  KTKSGVEVGAFATKAH SQAALDFPLDIAIRSIEFYEDYYQTPYPLP
Lb. lactis CVDEPEAKATFSLALKFDE HEGETVLANMPE    DRVENG  VHYFKETVRMSSYLVAFAFGEMRSLTT  HTKSGVLIGVYSTQAH TEKELTFSLDIAKRAIEFYEDFYQTPYPLP
S. cerevis CFDEPNLKASFAITLVSVP SLTHLSNMDVKN     EYVKDGKKVTLFNTTPKMSTYLVAFIVAELKYVES  K NFRIPVRVYATPGN EKHG QFAADLTAKTLAFFEKTFGIQYPLP
man        CFDEPAMKAEFNITLIHPK DLTALSNMLPKGPSTPLPEDPNWNVTEFHTTPKMSTYLLAFIVSEFDYVEK  QASNGVLIRIWARPSAIAAGHGDYALNVTGPILNFFAGHYDTPYPLP
rat        CFDEPAMKASFNITLIHPN NLTALSNMLPKDSRT LQEDPSWNVTEFHPTPKMSTYLLAYIVSEFKYVEA  VSPNRVQIRIWARPSAIDEGHGDYALQVTGPILNFFAQHYNTAYPLE
rabbit     CFDEPASKATFNITLIHPR DYTALSNMLPRSSTA LPEDPNWTVTEFHTTPKMSTYLLAYIVSEFTNIEA  QSPNNVQIRIWARPSAISEGHGQYALNVTGPILNFFANHYNTPYPLE
mouse      CFDEPNKKSTYSISIIHPK EYSALSNM PEEKSE MVDD NWKKTTFVKSVPMSTYLVCFAVHRFTAIER  KSRSGKPLKVYVQPH QKETAEYAANITQAVFDYFEDYFAMEYALP
E. coli    YLDRPDVLARFTTKIIADKIKYPFLLSNGNRVAQGELENGRHW  VQWQDFPKPCYLFALVAGDFDVLRDTFTTRSGREVALELYVDRGHLDRAPWAMTSLKNSMKWDEERFGLEYDLD

Lc. lactis HSWHIALPDFSAGAMENWGCITYREVCHLVDPENATIQSKQYVATVIAHELAHQWFGDLVTMQWWDDLWLNESFANNMEYVCMDALEPSWNVWESFSISEANMALNRDATDGVQSV   H
Lc. cremor HSWHIALPDFSSGAMENWGCITYREVCHLVDPENATIQSKQYVATVIAHELAHQWFGDLVTMQWWDDLWLNESFANNMEYVCMDALEPSWNVWESFSISEANMALNRDATDGVQSV   H
Lb. lactis QSLQLALPDFSAGAMENWGLVTYREAYLLLDPDNTTLEMKKLVATVVTHELAHQWFGDLVTMEWWDNLWLNESFANMMEYLSVDHLEPNWHIWEMFQTSEAAAALTRDATDGVQSV   H
S. cerevis KMDNVAVHEFSAGAMENWGLVTYRVVDLLLDKDNSTLDRIQRVAEVVQHELAHQWFGNLVTMDWWEGLWLNEGFATWMSWYSCNEFQPEWKVWEQYVTDTLQHALSLDSLRSSHPI   H
man        KSDQIGLPDFNAGAMENWGLVTYRENSLLFDPLSSSSSNKERVVTVIAHELAHQWFGNLVTIEWWNDLWLNEGFASYVEYLGADYAEPTWNLKDLMVLNDVYRVMAVDALASSHPLSTPA
rat        KSDQIALPDFNAGAMENWGLVTYRESALVFDPQSSSISNKERVVTVIAHELAHQWFGNLVTVDWWNDLWLNEGFASYVEFLGADYAEPTWNLKDLIVLNDVYRVMAVDALASSHPLSSPA
rabbit     KSDQIGLPDFNAGAMENWGLVTYRESALLFDPLVSSISNKERVVTVIAHELAHQWFGNLVTVDWWNDLWLNEGFASYVEYLGADYAEPTWNLKDLIVLNFLHSVMAVDALASSHPLSSPA
mouse      KLDKIAIPDFGTGAMENWGLVTYRETNLLYDPLLSASSNQORVASVVAHELVHQWFGNTVIMDWWDDLWLNEGFASFFEFLGVNHAEKDWQMLSQVLLEDVFPVQEDDSLMSSHPV   V
E. coli    IYMIVAVDFFNMGAMENKGLNIFNSKYVLARTDTATDKDYLDIERVIGHEYFHNWTGNRVTCRDWFQLSLKEGLTVFRD      QEFSSDLGSRAVNRINNVRTMRGLQFAEDASPM AHP
                                                     :::ZA::Z:::
```

Figure 4

```
                              *          *                 *   *   *                                    *    *
Lc. lactis   VEVTHPDEIGILFDPAIVYAKGSRLHVMLRKXLGDEDFAAGLALYFKRHQYGNTVGDNLWDALAEVSGKD    VAAFMHSVVNQPGYPVVT  AEVVDDTLILSQKQFFVGEGVDK
Lc. cremor   VEVTHPDEIGILFDPAIVYAKGSRLHVMLRKXLGDEDFAAGLALYFKRHQYGNTVGDNLWDALAEVSGKD    VAAFMHSVVNQPGYPVVT  AEVVDDTLILSQKQFFVGEGVDK
Lb. lactis   VEVNDPAEIDALFDGAIVYAKGSRMLVMVRSLLGDEALRKGLKRYFDKHKFGHAAGDDLWDALSTATDLN    IGEIMHTVLDQPGYPVVN  AFVEDGHLKLTQKQFFIGEGKEV
S. cerevis   VPVKKADEINQIFD AISYSKGASLLRMISKWLGEETFIKGVSQYLNKFKYGHAKTEDLWDALADASGKE    VRSVWNIWTKKVGFPVISVSEDGNGKITFRQNRYLSTADVKP
man          SEINTPAQISELFD AISYSKGASVLRMLSSFLSEDVFKQGLASYLHTFAYQNTIYLNLWDHLQEAVNNR SIQLPTTERDIMNRWTLQMGFPVIT  VDTSTGTLSQEHFLLDPDSNV
rat          NEVNTPAQISELFD SITYSKGASVLRMLSSFLTEDLFKKGLSSYLHTFQYSNTIYLDLWEHLQQAVDSQTAIKLPASVSTIMDRWILQMGFPVIT  VNTSTGEIYQEHFLLDPTSKP
rabbit       DEVNTPAQISELFD SITYSKGASVLRMLSSFLTEDLFKEGLASYLHTFAYQNTIYLDLWEHLQQAVNSQSAIQLPASVRDIMDRWILQMGFPVVT  VNTTNGIISQHHFLLDPTSRV
mouse        VTVSTPAEITSVFD GISYSKGASILRHLQDWITPEKFQKGQIYLKKFQFANAKTSDFWDSLQEASN  LP VKEVWDTWTSQMGYPVVT  VSGRQ NITQKRFLLDSKADP
E. coli      IRPDMVIEHMNFYTLTV YEKGAEVIRMIHTLLGEENFGKGMQLYFERHDGSAATCDDFVQANEDASNVD    LSHFRRWYSQSGTP1VT  VKDDYNPETEQYTLTISQRT


                  *
Lc. lactis   GR      LMNV PLNTKWTGLPDL LSSEKVEIPGFAALKTKNWGKALFLNDANWAHYIIDYKGALL    TDLLSEVESLENVTKFQILQDRKLLAKAGVISTADVVNILPSFTNEE
Lc. cremor   GR      LMNV PLNTKWTGLPDL LSSEKVEIPGFAALKTKNWGKALFLNDANWAHYIIDYKGALL    TDLLSEVESLENVTKFQILQDRKLLAKAGVISTADVVNILPSFTNEE
Lb. lactis   GR      KWEI PLNANFKA PKI MSDVELDLGDYQALRA EAGHALRLWVGNWSHFIVKYDQTLM    DDIMKEAKDLDPVSQLQLLQDLRLLAEGKQASTADVVPVLELFKHSE
S. cerevis   DE      DKTIYPVFLAKLTKNGV DSSVVLSERSKTIELEDPTFFKVWSEQSGYITSYTDERW    AKLGQQADLLSVEDRVGLVADVKTLSASGYTSTNFLMLVSKWNEK
man          TR PSEFNYWLIV PITSIRDGROQODYWLMD VRAQNDLFSTSG NEWVLLNLWVTGYYRVNYDEENWRKIQTGLQRDHSAINDAFNLASAHKVPTLALWNTLFLIEER
rat          TR PSDFNYLWIV PIPYLKNGKEDH YWLET EKWQSAEFQTSS NEWLLLWINVTGYYQVNYDEGNWRKIQNGLQTDLSVIPVINRAQIIHDSFNLASAGKLSITLPLSNTLFLASET
rabbit       TR PSDFNYLWIV PVSSHRNGVQQQEFWLEGVEQTQNSLFRVEGDNNWILAHLWVTGYYQVNYDEGNWKKLQTGLQTNPSVIPVINRAQIIHDAFNLASAGKVPTLALDNTLFLIRET
mouse        SQPPSELGYTWNI PVRWADWDNSRITVY NRLDKGGITLNANLSCDAFLKIHPDHIGFYRVWYEGGTWDWIAEALSSNHTRFSAADRSSFIDDAFALARAQLLWYKIALNLTNYLKSEE
E. coli      PATPDQAEKQPLHIPFAIELYDNEGKVIPLQKGGHPVNSVLNVTQAEGTFVFD  NVYFQP  VPALLCEFSAPVKLEYKWSDQQLTFLMKHARNDFSRWDAAQSLLATYIKLNVARHQ


Lc. lactis   SYLVNTGLSQLISELE  LFVDEDSETEKAFQSLVGKLFAKNYARL    GWDKVAGESAGDESLRGIVLSKTLYSEWADAKTKASQIFAAH KE NLASIPAD IRPIVLNNEIKTTNS
Lc. cremor   SYLVNTGLSQLISELE  LFVDEDSETEKAFQSLVGKLFAKNYARL    GWDKVAGESAGDESLRGIVLSKTLYSEWADAKTKASQIFATH KE NLASIPAD IRPIVLNNEIKTTNS
Lb. lactis   SHIVNDALYTTADKLR  QFAPAGSEADKWLRALYNDLSKDQVARL    GWLPKAGESDED IQTRPYVLSASLYGRNADSEKQAHEIYVEY AD KLAELSAD IRPYVLINEVENYGS
S. cerevis   SFVVWDQIINSISSMKSTWLFEPKETQDALDNFTKQLISGMTHHL    GWEFKSSDSFSTQRLKVTMFGWACAARDADVEKAALKMFTDYCSG NKEAIPAL IKPIVFNTVARVGGA
man          QYMPWEAALSSLSYFKLMF  DRSEVYGPMKWYLKKQVTPLFIHFRWNTNNKREIP ENLWDGYSEVNAISTACSNGVPECEEMVSGLFKQWENPNNNPIHPN LRSTVYCNAIAQ GG
rat          EYMPWEAALSSLNYFKLMF  DRSEVYGPMKRYLKKQVTPLFAYFKIKTNNWLDRP PTLMEQYNEINAISTACSNGIQECETLVSDLFKQWDDPSNNPIHPN LRSTVYCNAIA  SC
rabbit       DFLPWERVISSVSYIISMF  EDDRELYPMIETYFGQVKPVADLL    GW QDT GSHITKLLRASILGFACKWGDREALGNASQLFDSWLKGSASIPV N LRLLVYRYGMQNSGN
mouse        QGQPLSLPVHVADAFRAVLLDEKIDPALAAEILTLPSVWEHAELF    DIIDPIAIAEVREALTRTLATELADELLAIYNANYQSEYRVEHEDIAKRTLRWACLRFLAF GETHLADV


Lc. lactis   AELVKTYRETYIKTSLQEFKRELEGAVALIKDEKVIAELLESSFKNADIVKPQ    DIAFSWFY LLRNDFSDDAAWAWEKANWAF    LEEKLGGDMS YDKFVIYPGNTFKTAD KLA
Lc. cremor   AELVKTYRETYIKTSLQEFKRELEGAVALIKDEKVIAELLESSFKNADIVKPQ    DIAFSWFY LLRNDFSDDAAWAWEKANWAF    LEEKLGGDMS YDKFVIYPGNTFKTAD KLA
Lb. lactis   SELTDKLIGLYQATSDPSFKWDLEAAIVKSKDEGELKKIVSWFKNAEIVKPQ    DLR GWFSGVLSNPAGEQLAWDWIRDEWAW    LEKTVGGDME FATFITVISRVFKTKE RYD
S. cerevis   ENYEKVYK IYLDFISNDEKLAALRSLGRFKEPKLLERTLGYLFDGTVLN Q    DIYIP MQGWRAHQEGVEALWWVKKNWDE    LVKRLPPGLSMLGSVVTLGTSGFTSMQ KID
man          EEWDFAWEQFRNATLVNEADKLRAALACSKELWILNRYLSYTLNPDLIRKQ    DATST IISITNNVIGQGLVWDFVQSNWKK    PFNDYGGGSFSFSFANLIQGVTRRFSTEY KID
rat          EEAWNFAWATWPERFLVNEADKLRSAVGRSNEWILNRYLSYTLNPDYIRKQ    DATST IVSIANNVVGQTLVWDFVRSNWKK    LFEDYGGGSFSFANLIQGVTRRFSSEF ELQ
rabbit       EREWDFAWEQFRNATLVNEADKLRSALACSNEVWILNRYLSYTLNPDYIRKQ    DATST INSIASNVIGQTLVWDFVQSNWKK    LFEDFGGGSFSFANLIRAVTRRFSTEY ELQ
mouse        EAAWNYTLEQYQKTSLAQEKEKLLYGLASVKDVKLLARYLEMLKDPWIIKTQ    DVFTV IRYISYNSYGKTNAWNWIQLWHDY    LVSRFTIND RYLGRIVTIAEPFNTEL QLW
E. coli      LVSKQFHEAKNMTDALAALSAAVAAQLPCRDA LWQEYDDKWHQNGLVWDKWFILQATSPAANVLETVRGLLQHRSFTMSNPNRIRSLIGAFAGSNPAAFHAEDGSGYLFLVEMLTDLN


Lc. lactis   EYKAFFEPKLEN  QGLKRSIEMAIKQITARVALIDSQKAAVDKAITDIAEKL
Lc. cremor   EYKAFFEPKLEN  QGLKRSIEMAIKQITARVALIDSQKAAVDKAITDIAEKL
Lb. lactis   EYNAFFTDKESN  MLLNREIKWDRKVIANRVDLIASEQADVNAAVAAALQK
S. cerevis   EIKKFFATK ST  KGFDQSLAQSLDTITSKAQWVNRDRDVVNKYLKENGTY
man          QLEQFKDNEETGFGSGTRALEQALEKTKANIKWVKENKEVVLQWFTENS
rat          QLEQFKEDNSATGFGSGTRALEQALEKTKANIKWVKENKDVVLKWFTEN
rabbit       QLEQFRLNWLDTGFGSGTRALEQALEQTKANIKWQENKEAVLAWFTANSA
mouse        QMQSFFAKYPNA  GAGAKPREQVLETVKKNIEWLNVNRQSIREWFASLP
E. coli      SRNPQVASRLIEPLIRLKRYDAKRQEKMRAALEQLKGLENLSGDLYEKITKALA
```

Figure 4 (continued 1)

36

Figure 5

Figure 6

European Patent Office

**EUROPEAN SEARCH REPORT**

Application Number

EP 94 40 1497

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | JOURNAL OF DAIRY SCIENCE, vol.74, 1991, CHAPAIGN, ILLINOIS US pages 29 - 45 N. KHALID ET AL 'Peptide hydrolases of Lactobacillus helveticus and Lactobacillus delbrueckii ssp. bulgaricus' * page 36, left column - right column, paragraph 1; tables 1,5 * * abstract * | 1-18 | C12N15/57 C12N9/52 C12N1/21 A23C19/032 C12P21/06 |
| Y | APPL. MICROBIOL. BIOTECHNOL., vol.39, May 1993 pages 204 - 210 C. NOMAKOWSKI ET AL 'Cloning of peptidase genes from Lactobacillus helveticus CNRZ 32' * the whole document * | 1-18 | |
| Y | GENE., vol.113, 1992, AMSTERDAM NL pages 107 - 112 P. STROMAN 'Sequence of a gene (lap) encoding a 95.3-kDa aminopeptidase from Lactococcus lactis ssp. cremoris Wg2' * the whole document * | 1-18 | **TECHNICAL FIELDS SEARCHED** (Int.Cl.6) C12N A23C |
| Y | FEBS LETTERS., vol.306, no.1, July 1992, AMSTERDAM NL pages 9 - 16 P. TAN ET AL 'Characterization of the Lactococcus lactis pepN gene encoding an aminopeptidase homologous to mammalian aminopeptidase N' * the whole document * | 1-18 | |

-/--

The present search report has been drawn up for all claims

| Place of search | Date of completion of the search | Examiner |
|---|---|---|
| THE HAGUE | 14 October 1994 | Van der Schaal, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

# EUROPEAN SEARCH REPORT

Application Number

EP 94 40 1497

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | INT. DAIRY JOURNAL, vol.1, 1991 pages 51 - 66 W. BOCKELMANN AND M. FOBKER 'Purification and characterization of the X-prolyl-dipeptidyl-aminopeptidase from Lactobacillus delbrueckii subsp. bulgaricus and Lactobacillus acidophilus' * abstract * | 10,11, 14-18 | |
| Y | EP-A-0 522 203 (PROBICOM RESEARCH B.V.) 13 January 1993 * abstract * | 14-18 | |
| P,Y | CHEMICAL ABSTRACTS, vol. 120, no. 19, 9 May 1994, Columbus, Ohio, US; abstract no. 237307, E. MEYER-BARTON ET AL 'Cloning and sequence analysis of the X-prolyl-dipeptidyl-aminopeptidase gene (pepX) from Lactobacillus delbrueckii ssp. lactis DSM7290' page 251 ; * abstract * & APPL. MICROBIOL. BIOTECHNOL., vol.40, no.1, October 1993 pages 82 - 89 | 1-18 | |

-/--

**TECHNICAL FIELDS SEARCHED (Int.Cl.6)**

The present search report has been drawn up for all claims

| Place of search | Date of completion of the search | Examiner |
|---|---|---|
| THE HAGUE | 14 October 1994 | Van der Schaal, C |

| European Patent Office | EUROPEAN SEARCH REPORT | Application Number EP 94 40 1497 |
|---|---|---|

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,Y | CHEMICAL ABSTRACTS, vol. 119, no. 21, 22 November 1993, Columbus, Ohio, US; abstract no. 220172, E. TSAKALIDOU ET AL 'A comparative study: aminopeptidase activities from Lactobacillus delbrueckii ssp. bulgaricus and Streptococcus thermophilus' page 407 ; * abstract * & JOURNAL OF DAIRY SCIENCE, vol.76, no.8, August 1993, CHAPAIGN, ILLINOIS US pages 2145 - 2151 --- | 1-18 | |
| P,X | EUR. J. BIOCHEMISTRY, vol.217, October 1993 pages 105 - 114 J. KLEIN ET AL 'Cloning, DNA sequence analysis and partial characterization of pepN, a lysyl aminopeptidase from Lactobacillus delbrückii ssp. lactis DSM7290' * the whole document * ----- | 1-18 | TECHNICAL FIELDS SEARCHED (Int.Cl.6) |

The present search report has been drawn up for all claims

| Place of search | Date of completion of the search | Examiner |
|---|---|---|
| THE HAGUE | 14 October 1994 | Van der Schaal, C |

SEQUENCE LISTING

(1) GENERAL INFORMATION:

    (i) APPLICANT:
        (A) NAME: COMMUNAUTE ECONOMIQUE EUROPEENNE
        (B) STREET: BATIMENT JEAN MONNET - PLATEAU DU KIRCHBERG
        (C) CITY: LUXEMBOURG
        (D) STATE: LUXEMBOURG
        (E) COUNTRY: LUXEMBOURG
        (F) POSTAL CODE (ZIP): L2920

    (ii) TITLE OF INVENTION: LYS-AMINOPEPTIDASE PepN FROM LACTOBACILLUS
        DELBRUCKII SSP. LACTIS, NUCLEIC ACIDS CODING FOR IT, AND
        ITS USE IN FERMENTATION PROCESSES

    (iii) NUMBER OF SEQUENCES: 2

    (iv) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25 (EPO)

    (vi) PRIOR APPLICATION DATA:
        (A) APPLICATION NUMBER: GB 93-13586.1
        (B) FILING DATE: 01-JUL-1993

(2) INFORMATION FOR SEQ ID NO: 1:

    (i) SEQUENCE CHARACTERISTICS:
        (A) LENGTH: 3122 base pairs
        (B) TYPE: nucleic acid
        (C) STRANDEDNESS: double
        (D) TOPOLOGY: linear

    (ii) MOLECULE TYPE: DNA (genomic)

    (ix) FEATURE:
        (A) NAME/KEY: CDS
        (B) LOCATION: 316..2844

    (xi) SEQUENCE DESCRIPTION: SEQ ID NO: 1:

```
GATAGGGCTG AAATTATCAT TTTAAGCGCT TTAAATTAGC TATACAGATA AGTAACATTA        60

GTAACAATTG TCAAGAGACT GCAATAAAAG GAAAAGGCCA GCTGCTAGAC TGGTCTTTTA       120

CATATGCAAT TATTTCAAAA ATGGAATTAA TTTCCGTTAG AAACTGAATT GTCTGATCGA       180

GTTCAAGGGG CTGAGGTAGA CTGCAAACAG ATATTTTGCG TTAAATGGGC TTTATTTAGC       240

CTTTTTTGCT AGAATAGAGA AGTGTGAATA CAATATACGC GAGGAAAAAT CAGACGCGGA       300
```

```
TTATAGGAGG AAACA ATG GCT GTT AAG CGT TTT TAC GAA ACA TTC CAT CCA          351
            Met Ala Val Lys Arg Phe Tyr Glu Thr Phe His Pro
             1               5                   10

GAT CAC TAC GAT CTA TAC ATC GAC GTT GAC CGG GCA GCA AGA TCT TTC          399
Asp His Tyr Asp Leu Tyr Ile Asp Val Asp Arg Ala Ala Arg Ser Phe
         15               20              25

TCA GGG ACT TCC ACC ATC CAT GGT GAA ATC CAG GAA GAA ACT GTC TTG          447
Ser Gly Thr Ser Thr Ile His Gly Glu Ile Gln Glu Glu Thr Val Leu
         30               35              40

GTC CAC CAA AAG TAC ATG ACC ATC AGC AAG GTC ACT GTT GAC GGC AAG          495
Val His Gln Lys Tyr Met Thr Ile Ser Lys Val Thr Val Asp Gly Lys
 45              50              55                  60

GAA GTG CCG TTT ACT TTT GGC GAC GAC TTT GAA GGC ATC AAG ATC GAA          543
Glu Val Pro Phe Thr Phe Gly Asp Asp Phe Glu Gly Ile Lys Ile Glu
             65              70                  75

GCC GGC AAG ACT GGG GAA GCG GTC ATC GCG ATC GAC TAC TCA GCG CCT          591
Ala Gly Lys Thr Gly Glu Ala Val Ile Ala Ile Asp Tyr Ser Ala Pro
             80              85                  90

TTG ACT GAC ACT ATG ATG GGG ATC TAC CCG TCC TAC TAC CAA GTT GAT          639
Leu Thr Asp Thr Met Met Gly Ile Tyr Pro Ser Tyr Tyr Gln Val Asp
             95              100             105

GGC GTC AAG AAG GAA CTG ATC GGG ACC CAG TTT GAA ACT ACT TTT GCC          687
Gly Val Lys Lys Glu Leu Ile Gly Thr Gln Phe Glu Thr Thr Phe Ala
    110              115             120

CGG GAA GCC TTC CCA TGT GTT GAC GAA CCA GAA GCT AAG GCA ACC TTC          735
Arg Glu Ala Phe Pro Cys Val Asp Glu Pro Glu Ala Lys Ala Thr Phe
125              130             135             140

TCC CTC GCC CTC AAG TTT GAC GAA CAC GAA GGC GAA ACT GTT TTG GCC          783
Ser Leu Ala Leu Lys Phe Asp Glu His Glu Gly Glu Thr Val Leu Ala
             145             150             155

AAC ATG CCA GAA GAC CGG GTA GAA AAC GGC GTG CAC TAC TTT AAG GAG          831
Asn Met Pro Glu Asp Arg Val Glu Asn Gly Val His Tyr Phe Lys Glu
             160             165             170

ACT GTC CGC ATG TCC AGC TAC CTG GTT GCT TTT GCC TTT GGT GAA ATG          879
Thr Val Arg Met Ser Ser Tyr Leu Val Ala Phe Ala Phe Gly Glu Met
         175             180             185

CGG TCA TTG ACC ACC CAC ACC AAG AGC GGG GTC TTG ATC GGG GTT TAC          927
Arg Ser Leu Thr Thr His Thr Lys Ser Gly Val Leu Ile Gly Val Tyr
    190              195             200

TCA ACT CAA GCC CAC ACT GAA AAG GAA CTG ACC TTC TCC TTG GAC ATT          975
Ser Thr Gln Ala His Thr Glu Lys Glu Leu Thr Phe Ser Leu Asp Ile
205              210             215                 220
```

```
GCC AAG CGG GCA ATT GAA TTC TAC GAA GAC TTT TAC CAA ACT CCA TAT        1023
Ala Lys Arg Ala Ile Glu Phe Tyr Glu Asp Phe Tyr Gln Thr Pro Tyr
                225                 230                 235

CCG CTG CCG CAA TCA CTG CAG CTG GCC CTG CCT GAC TTC TCA GCC GGT        1071
Pro Leu Pro Gln Ser Leu Gln Leu Ala Leu Pro Asp Phe Ser Ala Gly
                240                 245                 250

GCC ATG GAA AAC TGG GGT CTG GTA ACC TAC CGG GAA GCC TAC CTG CTT        1119
Ala Met Glu Asn Trp Gly Leu Val Thr Tyr Arg Glu Ala Tyr Leu Leu
                255                 260                 265

TTG GAC CCG GAC AAC ACC ACT TTG GAA ATG AAG AAG CTG GTT GCG ACT        1167
Leu Asp Pro Asp Asn Thr Thr Leu Glu Met Lys Lys Leu Val Ala Thr
    270                 275                 280

GTG GTG ACC CAC GAA CTG GCC CAC CAA TGG TTC GGT GAC CTG GTA ACC        1215
Val Val Thr His Glu Leu Ala His Gln Trp Phe Gly Asp Leu Val Thr
285                 290                 295                 300

ATG GAA TGG TGG GAC AAC CTC TGG CTG AAC GAA AGT TTC GCC AAC ATG        1263
Met Glu Trp Trp Asp Asn Leu Trp Leu Asn Glu Ser Phe Ala Asn Met
                305                 310                 315

ATG GAA TAC CTG TCA GTT GAC CAC CTG GAA CCT AAC TGG CAC ATC TGG        1311
Met Glu Tyr Leu Ser Val Asp His Leu Glu Pro Asn Trp His Ile Trp
                320                 325                 330

GAA ATG TTC CAG ACT TCT GAA GCA GCG GCT GCC TTG ACC CGG GAT GCA        1359
Glu Met Phe Gln Thr Ser Glu Ala Ala Ala Ala Leu Thr Arg Asp Ala
                335                 340                 345

ACC GAC GGG GTA CAG TCA GTG CAC GTG GAA GTT AAT GAC CCG GCT GAA        1407
Thr Asp Gly Val Gln Ser Val His Val Glu Val Asn Asp Pro Ala Glu
    350                 355                 360

ATC GAC GCC CTC TTT GAC GGG GCC ATC GTT TAC GCC AAG GGG TCA AGA        1455
Ile Asp Ala Leu Phe Asp Gly Ala Ile Val Tyr Ala Lys Gly Ser Arg
365                 370                 375                 380

ATG CTG GTC ATG GTC CGG TCA CTT TTG GGC GAT GAA GCC TTG AGA AAG        1503
Met Leu Val Met Val Arg Ser Leu Leu Gly Asp Glu Ala Leu Arg Lys
                385                 390                 395

GGC TTG AAG CGC TAC TTT GAC AAG CAC AAG TTT GGC AAC GCG GCA GGT        1551
Gly Leu Lys Arg Tyr Phe Asp Lys His Lys Phe Gly Asn Ala Ala Gly
                400                 405                 410

GAC GAT CTC TGG GAT GCC TTG TCA ACG GCC ACT GAC TTG AAC ATT GGG        1599
Asp Asp Leu Trp Asp Ala Leu Ser Thr Ala Thr Asp Leu Asn Ile Gly
                415                 420                 425

GAA ATC ATG CAC ACT TGG CTG GAC CAG CCA GGC TAC CCA GTG GTG AAT        1647
Glu Ile Met His Thr Trp Leu Asp Gln Pro Gly Tyr Pro Val Val Asn
    430                 435                 440
```

```
GCT TTT GTT GAG GAC GGC CAC TTG AAG CTG ACT CAG AAG CAA TTC TTC    1695
Ala Phe Val Glu Asp Gly His Leu Lys Leu Thr Gln Lys Gln Phe Phe
445             450             455             460

ATC GGT GAA GGC AAG GAA GTC GGC CGC AAG TGG GAA ATT CCG CTT AAC    1743
Ile Gly Glu Gly Lys Glu Val Gly Arg Lys Trp Glu Ile Pro Leu Asn
                465             470             475

GCT AAC TTC AAG GCA CCG AAG ATC ATG TCA GAC GTT GAA CTT GAC CTG    1791
Ala Asn Phe Lys Ala Pro Lys Ile Met Ser Asp Val Glu Leu Asp Leu
                480             485             490

GGT GAC TAC CAG GCT CTG CGG GCA GAA GCC GGC CAC GCT CTG CGC TTG    1839
Gly Asp Tyr Gln Ala Leu Arg Ala Glu Ala Gly His Ala Leu Arg Leu
                495             500             505

AAC GTG GGC AAC AAC TCC CAC TTC ATC GTG AAG TAC GAC CAG ACT TTG    1887
Asn Val Gly Asn Asn Ser His Phe Ile Val Lys Tyr Asp Gln Thr Leu
    510             515             520

ATG GAC GAC ATC ATG AAG GAA GCC AAG GAC TTG GAT CCA GTT TCC CAA    1935
Met Asp Asp Ile Met Lys Glu Ala Lys Asp Leu Asp Pro Val Ser Gln
525             530             535             540

TTG CAA TTG CTG CAA GAC CTG CGG CTT TTG GCA GAA GGC AAG CAG GCT    1983
Leu Gln Leu Leu Gln Asp Leu Arg Leu Leu Ala Glu Gly Lys Gln Ala
                545             550             555

TCA TAC GCT GAC GTG GTA CCA GTT CTG GAA CTC TTC AAG AAC TCA GAA    2031
Ser Tyr Ala Asp Val Val Pro Val Leu Glu Leu Phe Lys Asn Ser Glu
                560             565             570

AGC CAC ATT GTC AAC GAT GCT CTG TAC ACG ACT GCT GAT AAG CTG CGG    2079
Ser His Ile Val Asn Asp Ala Leu Tyr Thr Thr Ala Asp Lys Leu Arg
        575             580             585

CAA TTT GCC CCA GCC GGC AGT GAA GCT GAC AAG AAC CTG CGG GCT CTG    2127
Gln Phe Ala Pro Ala Gly Ser Glu Ala Asp Lys Asn Leu Arg Ala Leu
    590             595             600

TAC AAC GAC TTG TCC AAG GAC CAA GTT GCC CGT TTG GGC TGG CTG CCT    2175
Tyr Asn Asp Leu Ser Lys Asp Gln Val Ala Arg Leu Gly Trp Leu Pro
605             610             615             620

AAG GCA GGG GAA AGC GAT GAA GAC ATT CAG ACC CGG CCA TAC GTT TTG    2223
Lys Ala Gly Glu Ser Asp Glu Asp Ile Gln Thr Arg Pro Tyr Val Leu
                625             630             635

TCT GCT AGC CTT TAC GGC CGC AAC GCT GAT TCA GAA AAG CAA GCC CAC    2271
Ser Ala Ser Leu Tyr Gly Arg Asn Ala Asp Ser Glu Lys Gln Ala His
                640             645             650

GAA ATC TAC GTG GAA TAC GCT GAT AAG TTG GCA GAA CTG TCC GCT GAT    2319
Glu Ile Tyr Val Glu Tyr Ala Asp Lys Leu Ala Glu Leu Ser Ala Asp
        655             660             665
```

```
ATC CGG CCA TAC GTT TTG ATC AAC GAA GTT GAA AAC TAC GGG TCA AGC        2367
Ile Arg Pro Tyr Val Leu Ile Asn Glu Val Glu Asn Tyr Gly Ser Ser
    670             675             680

GAA TTG ACT GAC AAG CTG ATT GGT TTG TAC CAG GCA ACC AGT GAC CCA        2415
Glu Leu Thr Asp Lys Leu Ile Gly Leu Tyr Gln Ala Thr Ser Asp Pro
685             690             695             700

TCA TTC AAG ATG GAC CTG GAA GCC GCG ATC GTG AAG AGC AAG GAC GAA        2463
Ser Phe Lys Met Asp Leu Glu Ala Ala Ile Val Lys Ser Lys Asp Glu
                705             710             715

GGC GAA CTG AAG AAG ATC GTT TCC TGG TTC AAA AAC GCT GAA ATC GTT        2511
Gly Glu Leu Lys Lys Ile Val Ser Trp Phe Lys Asn Ala Glu Ile Val
            720             725             730

AAG CCG CAG GAC TTG CGC GGC TGG TTC AGC GGC GTT TTG TCC AAC CCG        2559
Lys Pro Gln Asp Leu Arg Gly Trp Phe Ser Gly Val Leu Ser Asn Pro
        735             740             745

GCA GGT GAA CAG CTG GCC TGG GAC TGG ATC AGA GAC GAA TGG GCA TGG        2607
Ala Gly Glu Gln Leu Ala Trp Asp Trp Ile Arg Asp Glu Trp Ala Trp
    750             755             760

TTG GAA AAG ACG GTC GGC GGC GAC ATG GAA TTC GCT ACC TTC ATC ACT        2655
Leu Glu Lys Thr Val Gly Gly Asp Met Glu Phe Ala Thr Phe Ile Thr
765             770             775             780

GTC ATC TCC CGC GTC TTC AAG ACC AAG GAA CGC TAC GAC GAA TAC AAC        2703
Val Ile Ser Arg Val Phe Lys Thr Lys Glu Arg Tyr Asp Glu Tyr Asn
            785             790             795

GCC TTC TTT ACT GAC AAG GAA AGC AAC ATG CTG CTG AAC CGG GAA ATC        2751
Ala Phe Phe Thr Asp Lys Glu Ser Asn Met Leu Leu Asn Arg Glu Ile
            800             805             810

AAG ATG GAC CGG AAG GTC ATC GCT AAC CGG GTA GAC TTG ATT GCC AGC        2799
Lys Met Asp Arg Lys Val Ile Ala Asn Arg Val Asp Leu Ile Ala Ser
        815             820             825

GAA CAA GCT GAC GTC AAC GCC GCG GTT GCT GCT GCT TTG CAA AAG            2844
Glu Gln Ala Asp Val Asn Ala Ala Val Ala Ala Ala Leu Gln Lys
        830             835             840

TAATTGAATA GAGCATAAGA AAACTGTTTC CGCTGAGAGC TGGAAACAGT TTTTTTATGT      2904

ATTCAACTTG TCTGCAATCG GTTACAATAT AGATGTAAAT ACTATCGTAC ATTCTTTGAG      2964

GTAATAAAAT GAACAACGAT TTTAAAGATA TCATGCAAAA CAGAAAGTCT ATCCGGCACT      3024

ATGATTCCAG CGTGAAGATT TCCCGTGACG AATTGCTGGA AATCATTAAT GAATCTATCT      3084

CTGCTCCAAG TGCCTGCAAC CTGCAGTCCT GGAAGCTT                              3122
```

(2)  INFORMATION FOR SEQ ID NO: 2:

    (i)  SEQUENCE CHARACTERISTICS:
      (A)  LENGTH: 843 amino acids
      (B)  TYPE: amino acid
      (D)  TOPOLOGY: linear

    (ii)  MOLECULE TYPE: protein

    (xi)  SEQUENCE DESCRIPTION: SEQ ID NO: 2:

```
Met Ala Val Lys Arg Phe Tyr Glu Thr Phe His Pro Asp His Tyr Asp
 1               5                  10                  15

Leu Tyr Ile Asp Val Asp Arg Ala Ala Arg Ser Phe Ser Gly Thr Ser
            20                  25                  30

Thr Ile His Gly Glu Ile Gln Glu Glu Thr Val Leu Val His Gln Lys
            35                  40                  45

Tyr Met Thr Ile Ser Lys Val Thr Val Asp Gly Lys Glu Val Pro Phe
    50                  55                  60

Thr Phe Gly Asp Asp Phe Glu Gly Ile Lys Ile Glu Ala Gly Lys Thr
 65                  70                  75                  80

Gly Glu Ala Val Ile Ala Ile Asp Tyr Ser Ala Pro Leu Thr Asp Thr
                85                  90                  95

Met Met Gly Ile Tyr Pro Ser Tyr Tyr Gln Val Asp Gly Val Lys Lys
            100                 105                 110

Glu Leu Ile Gly Thr Gln Phe Glu Thr Thr Phe Ala Arg Glu Ala Phe
        115                 120                 125

Pro Cys Val Asp Glu Pro Glu Ala Lys Ala Thr Phe Ser Leu Ala Leu
    130                 135                 140

Lys Phe Asp Glu His Glu Gly Glu Thr Val Leu Ala Asn Met Pro Glu
145                 150                 155                 160

Asp Arg Val Glu Asn Gly Val His Tyr Phe Lys Glu Thr Val Arg Met
                165                 170                 175

Ser Ser Tyr Leu Val Ala Phe Ala Phe Gly Glu Met Arg Ser Leu Thr
            180                 185                 190

Thr His Thr Lys Ser Gly Val Leu Ile Gly Val Tyr Ser Thr Gln Ala
            195                 200                 205

His Thr Glu Lys Glu Leu Thr Phe Ser Leu Asp Ile Ala Lys Arg Ala
    210                 215                 220

Ile Glu Phe Tyr Glu Asp Phe Tyr Gln Thr Pro Tyr Pro Leu Pro Gln
225                 230                 235                 240

Ser Leu Gln Leu Ala Leu Pro Asp Phe Ser Ala Gly Ala Met Glu Asn
                245                 250                 255
```

```
Trp Gly Leu Val Thr Tyr Arg Glu Ala Tyr Leu Leu Leu Asp Pro Asp
        260                 265             270

Asn Thr Thr Leu Glu Met Lys Lys Leu Val Ala Thr Val Val Thr His
        275                 280             285

Glu Leu Ala His Gln Trp Phe Gly Asp Leu Val Thr Met Glu Trp Trp
    290                 295             300

Asp Asn Leu Trp Leu Asn Glu Ser Phe Ala Asn Met Met Glu Tyr Leu
305             310                 315             320

Ser Val Asp His Leu Glu Pro Asn Trp His Ile Trp Glu Met Phe Gln
            325                 330                 335

Thr Ser Glu Ala Ala Ala Ala Leu Thr Arg Asp Ala Thr Asp Gly Val
        340                 345             350

Gln Ser Val His Val Glu Val Asn Asp Pro Ala Glu Ile Asp Ala Leu
        355                 360                 365

Phe Asp Gly Ala Ile Val Tyr Ala Lys Gly Ser Arg Met Leu Val Met
    370                 375                 380

Val Arg Ser Leu Leu Gly Asp Glu Ala Leu Arg Lys Gly Leu Lys Arg
385             390                 395                 400

Tyr Phe Asp Lys His Lys Phe Gly Asn Ala Ala Gly Asp Asp Leu Trp
            405                 410                 415

Asp Ala Leu Ser Thr Ala Thr Asp Leu Asn Ile Gly Glu Ile Met His
        420                 425                 430

Thr Trp Leu Asp Gln Pro Gly Tyr Pro Val Val Asn Ala Phe Val Glu
        435                 440                 445

Asp Gly His Leu Lys Leu Thr Gln Lys Gln Phe Phe Ile Gly Glu Gly
    450                 455                 460

Lys Glu Val Gly Arg Lys Trp Glu Ile Pro Leu Asn Ala Asn Phe Lys
465                 470                 475                 480

Ala Pro Lys Ile Met Ser Asp Val Glu Leu Asp Leu Gly Asp Tyr Gln
            485                 490                 495

Ala Leu Arg Ala Glu Ala Gly His Ala Leu Arg Leu Asn Val Gly Asn
        500                 505                 510

Asn Ser His Phe Ile Val Lys Tyr Asp Gln Thr Leu Met Asp Asp Ile
    515                 520                 525

Met Lys Glu Ala Lys Asp Leu Asp Pro Val Ser Gln Leu Gln Leu Leu
    530                 535                 540

Gln Asp Leu Arg Leu Leu Ala Glu Gly Lys Gln Ala Ser Tyr Ala Asp
545             550                 555                 560
```

23

```
Val Val Pro Val Leu Glu Leu Phe Lys Asn Ser Glu Ser His Ile Val
            565             570             575

Asn Asp Ala Leu Tyr Thr Thr Ala Asp Lys Leu Arg Gln Phe Ala Pro
            580             585             590

Ala Gly Ser Glu Ala Asp Lys Asn Leu Arg Ala Leu Tyr Asn Asp Leu
            595             600             605

Ser Lys Asp Gln Val Ala Arg Leu Gly Trp Leu Pro Lys Ala Gly Glu
            610             615             620

Ser Asp Glu Asp Ile Gln Thr Arg Pro Tyr Val Leu Ser Ala Ser Leu
625             630             635             640

Tyr Gly Arg Asn Ala Asp Ser Glu Lys Gln Ala His Glu Ile Tyr Val
            645             650             655

Glu Tyr Ala Asp Lys Leu Ala Glu Leu Ser Ala Asp Ile Arg Pro Tyr
            660             665             670

Val Leu Ile Asn Glu Val Glu Asn Tyr Gly Ser Ser Glu Leu Thr Asp
            675             680             685

Lys Leu Ile Gly Leu Tyr Gln Ala Thr Ser Asp Pro Ser Phe Lys Met
            690             695             700

Asp Leu Glu Ala Ala Ile Val Lys Ser Lys Asp Glu Gly Glu Leu Lys
705             710             715             720

Lys Ile Val Ser Trp Phe Lys Asn Ala Glu Ile Val Lys Pro Gln Asp
            725             730             735

Leu Arg Gly Trp Phe Ser Gly Val Leu Ser Asn Pro Ala Gly Glu Gln
            740             745             750

Leu Ala Trp Asp Trp Ile Arg Asp Glu Trp Ala Trp Leu Glu Lys Thr
            755             760             765

Val Gly Gly Asp Met Glu Phe Ala Thr Phe Ile Thr Val Ile Ser Arg
            770             775             780

Val Phe Lys Thr Lys Glu Arg Tyr Asp Glu Tyr Asn Ala Phe Phe Thr
785             790             795             800

Asp Lys Glu Ser Asn Met Leu Leu Asn Arg Glu Ile Lys Met Asp Arg
            805             810             815

Lys Val Ile Ala Asn Arg Val Asp Leu Ile Ala Ser Glu Gln Ala Asp
            820             825             830

Val Asn Ala Ala Val Ala Ala Ala Leu Gln Lys
            835             840
```